# EUROPEAN PATENT APPLICATION

(11) **EP 4 570 803 A1**
(43) Date of publication of application: **18.06.2025**
(21) Application number: 23851779.1
(22) Date of filing: 07.08.2023
(51) Int. Cl.: C07D 495/04, A61P 35/00

(54) **SALT FORM AND CRYSTAL FORM OF 5,6-DIHYDROTHIENO[3,4-H]QUINAZOLINE COMPOUND AND PREPARATION METHOD THEREFOR**

(30) Priority: 08.08.2022 CN 202210948349
(71) Applicant: NovaOnco JS Therapeutics Co., Ltd., Xuzhou, Jiangsu 221132 (CN)
(72) Inventor: ZHANG, Dongkai, Shanghai 200131 (CN); XU, Yangyang, Shanghai 200131 (CN); SUN, Jikui, Shanghai 200131 (CN); WU, Wentao, Shanghai 200131 (CN); ZHANG, Yang, Shanghai 200131 (CN); CHEN, Shuhui, Shanghai 200131 (CN)
(74) Representative: Høiberg P/S
(86) International application number: PCT/CN2023/111516
(87) International publication number: WO 2024/032558

(57) **Abstract**

Disclosed are a salt form and a crystal form of a 5,6-dihydrothieno[3,4- *h*]quinazoline compound, a preparation method therefor, and use of the salt form and the crystal form in the preparation of medicaments for treating colorectal cancer and other solid tumors.

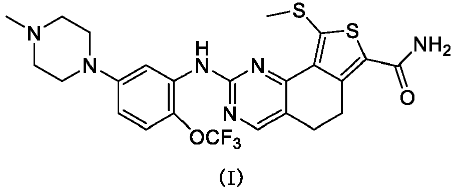

## Description

This application claims the benefit of priority to Chinese application No. 202210948349.2, filed August 8, 2022.

### Technical Field

The present application relates to a salt form and a crystalline form of a 5,6-dihydrothieno[3,4-*h* ]quinazoline compound and a preparation method thereof, as well as use of the salt form and the crystalline form in preparing drugs for treating colorectal cancer and other solid tumors.

### Background Art

Polo-like kinases (PLKs) are a class of highly conserved serine / threonine protein kinases, all of which have a highly homologous serine / threonine kinase domain at their N- termini and a characteristic domain (polobox domain, PBD) at their C -termini that regulates PLKs activity and subcellular dynamic localization. The PLKs family has many members, and there are four subtypes in the human body, namely PLK1, PLK2, PLK3 and PLK4, which play a vital role in the regulation of each phase of the cell cycle. Among these four family members, PLK1 is currently the most thoroughly studied. Therefore, PLK1 is a widely-watched target in tumor diagnosis and treatment.

Cardiff Oncology (formerly Trovagene) is developing onvansertib (PCM-075; NMS-P937; NMS-1286937; NMS-937) as a fumarate salt, an oral PLK-1 inhibitor, under license from Nerviano. Onvansertib is a potential oral cancer treatment for indications including metastatic colorectal cancer (mCRC), solid tumors, acute myeloid leukemia (AML), and metastatic castration-resistant prostate cancer. PLK1 is an effective therapeutic target that is overexpressed in most cancers, and Onvansertib is a novel, highly selective PLK1 inhibitor.

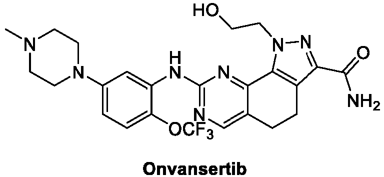

Onvansertib (NMS-1286937) is a highly selective PLK1 inhibitor currently under clinical development, with strong inhibitory effect on PLK1 kinase protein, and is currently conducting phase I or II clinical studies in the treatment of recurrent small cell lung cancer (SCLC) as a monotherapy, and in combination with SOC in the treatment of solid tumors such as KRAS-mutant metastatic colorectal cancer (mCRC), metastatic pancreatic cancer (mPDAC), and metastatic castration-resistant prostate cancer (mCRPC). Compared with KRASG12C inhibitors, PLK1 inhibitors have a higher response rate in CRC patients and are effective against all KRAS mutation subtypes. CRC is the third largest malignant tumor after lung cancer and breast cancer. The global market in 2018 was approximately US$ 25 billion, so it is possible to find highly active, highly selective, and metabolically stable small molecule PLK1 inhibitors for the treatment of tumors.

### Summary

The present application provides succinate, fumarate, benzenesulfonate, sulfate, phosphate, maleate, L-tartrate, methanesulfonate, L-malate, hydrochloride, citrate and L-aspartate of the compound of formula (I):

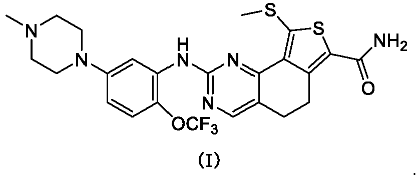

In some embodiments of the present application, the salt of the above compound is selected from: 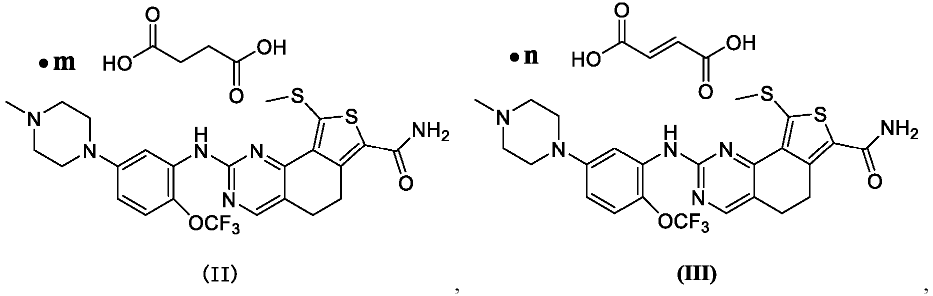 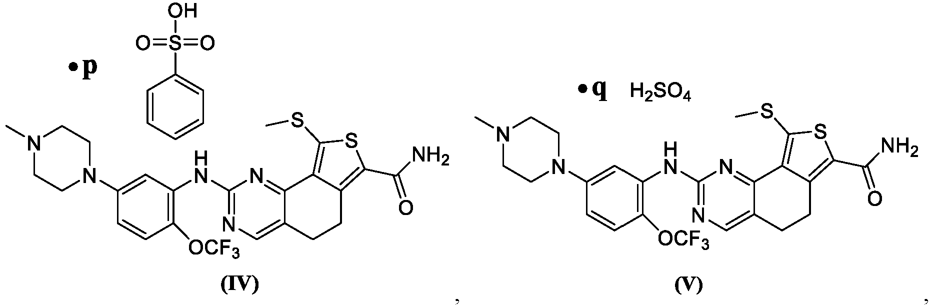 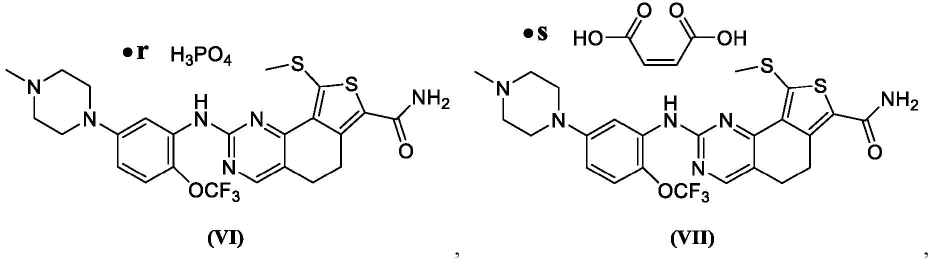 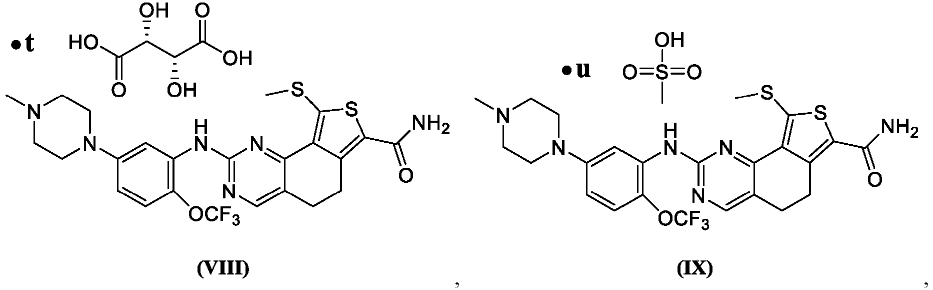 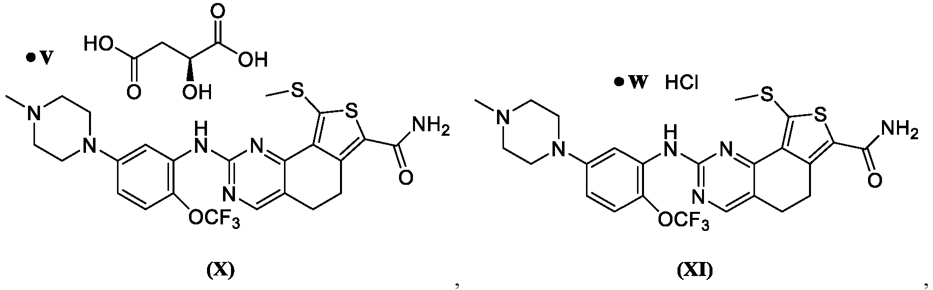 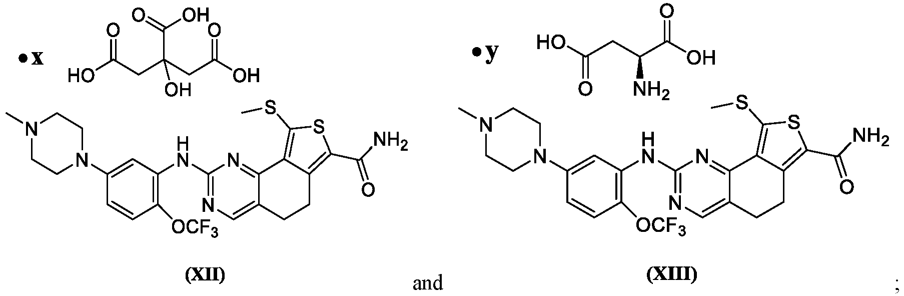 wherein m, n, p, q, r, s, t, u, v, w, x and y are independently selected from 0.5 to 3.0.

In some embodiments of the present application, in the above-mentioned salt of the formula, m, n, p, q, r, s, t, u, v, w, x and y are independently selected from 0.8 to 2.0.

In some embodiments of the present application, in the above-mentioned salt of the formula, m, n, p, q, r, s, t, u, v, w, x and y are independently selected from 0.8, 0.9, 1.0, 1.1, 1.2, 1.3, 1.4, 1.5, 1.6, 1.7, 1.8, 1.9 and 2.0.

In some embodiments of the present application, in the above-mentioned salt of the formula, m is 1.0, n is 1.0, p is 1.0, q is 1.0, r is 1.0, s is 1.0, t is 1.0, u is 1.0, v is 1.0, w is 1.0, x is 1.0, and y is 1.0.

In some embodiments of the present application, in the above-mentioned salt of the formula, m, n, p, q, r, s, t, u, v, w, x and y are independently selected from 1.0.

The present application provides a crystalline form A of a compound of formula (I), characterized in that its X-ray powder diffraction pattern has characteristic diffraction peaks at the following 2θ angles: 6.305±0.200°, 9.140±0.200°, 12.661±0.200°, and 19.637±0.200°,

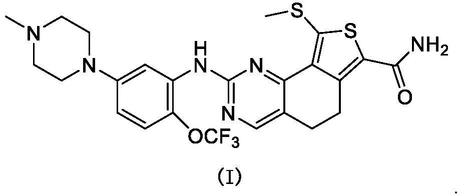

In some embodiments of the present application, the X-ray powder diffraction pattern of the above-mentioned crystalline form A has characteristic diffraction peaks at the following 2θ angles: 6.305±0.200°, 9.140±0.200°, 9.681±0.200°, 12.661±0.200°, 19.637±0.200°, 23.281±0.200°, and 24.892±0.200°.

In some embodiments of the present application, the X-ray powder diffraction pattern of the above-mentioned crystalline form A has characteristic diffraction peaks at the following 2θ angles: 6.305±0.200°, 9.140±0.200°, 9.681±0.200°, 12.661±0.200°, 14.139±0.200°, 19.637±0.200°, 23.281±0.200°, and 24.892±0.200°.

In some embodiments of the present application, the X-ray powder diffraction pattern of the above-mentioned crystalline form A has characteristic diffraction peaks at the following 2θ angles: 6.305±0.200°, 9.140±0.200°, 9.681±0.200°, 12.661±0.200°, 13.479±0.200°, 19.637±0.200°, 23.281±0.200°, and 24.892±0.200°.

In some embodiments of the present application, the X-ray powder diffraction pattern of the above-mentioned crystalline form A has characteristic diffraction peaks at the following 2θ angles: 6.305±0.200°, 9.140±0.200°, 9.681±0.200°, 12.661±0.200°, 13.479±0.200°, 14.139±0.200°, 16.523±0.200°, 18.385±0.200°, 19.637±0.200°, 21.161±0.200°, 23.281±0.200°, and 24.892±0.200°.

In some embodiments of the present application, the X-ray powder diffraction pattern of the above-mentioned crystalline form A has characteristic diffraction peaks at the following 2θ angles: 6.305±0.200°, 9.140±0.200°, 9.681±0.200°, 11.138±0.200°, 12.661±0.200°, 13.479±0.200°, 14.139±0.200°, 15.175±0.200°, 16.523±0.200°, 18.385±0.200°, 19.637±0.200°, 21.161±0.200°, 22.159±0.200°, 23.281±0.200°, 24.892±0.200°, and 26.582±0.200°.

In some embodiments of the present application, the X-ray powder diffraction pattern of the above-mentioned crystalline form A has characteristic diffraction peaks at the following 2θ angles: 6.305±0.200°, 9.140±0.200°, 19.637±0.200°, and/or 9.681±0.200°, and/or 11.138±0.200°, and/or 12.661±0.200°, and/or 13.479±0.200°, and/or 14.139±0.200°, and/or 15.175±0.200°, and/or 16.523±0.200°, and/or 18.385±0.200°, and/or 18.710±0.200°, and/or 19.904±0.200°, and/or 20.692±0.200°, and/or 21.161±0.200°, and/or 21.915±0.200°, and/or 22.159±0.200°, and/or 23.281±0.200°, and/or 24.892±0.200°, and/or 25.491±0.200°, and/or 26.582±0.200°, and/or 32.389±0.200°.

In some embodiments of the present application, the X-ray powder diffraction pattern of the above-mentioned crystalline form A has characteristic diffraction peaks at the following 2θ angles: 6.305±0.200°, 9.140±0.200°, 19.637±0.200°, and/or 9.681±0.200°, and/or 11.138±0.200°, and/or 12.661±0.200°, and/or 13.479±0.200°, and/or 14.139±0.200°, and/or 15.175±0.200°, and/or 16.523±0.200°, and/or 18.385±0.200°, and/or 18.710±0.200°, and/or 19.094±0.200°, and/or 19.904±0.200°, and/or 20.692±0.200°, and/or 21.161±0.200°, and/or 21.915±0.200°, and/or 22.159±0.200°, and/or 23.281±0.200°, and/or 23.630±0.200°, and/or 24.892±0.200°, and/or 25.491±0.200°, and/or 26.582±0.200°, and/or 32.389±0.200°.

In some embodiments of the present application, the X-ray powder diffraction pattern of the above-mentioned crystalline form A has characteristic diffraction peaks at the following 2θ angles: 6.305°, 9.140°, 9.681°, 11.138°, 12.661°, 13.479°, 14.139°, 16.523°, 18.385°, 19.637°, 19.904°, 21.161°, 22.159°, 23.281°, 24.892°, and 26.582°.

In some embodiments of the present application, the X-ray powder diffraction pattern of the above-mentioned crystalline form A has characteristic diffraction peaks at the following 2θ angles: 6.305°, 9.140°, 9.681°, 11.138°, 12.661°, 13.479°, 14.139°, 15.175°, 16.523°, 18.385°, 18.710°, 19.094°, 19.637°, 19.904°, 20.692°, 21.161°, 21.915°, 22.159°, 23.281°, 23.630°, 24.892°, 25.491°, 26.582°, and 32.389°.

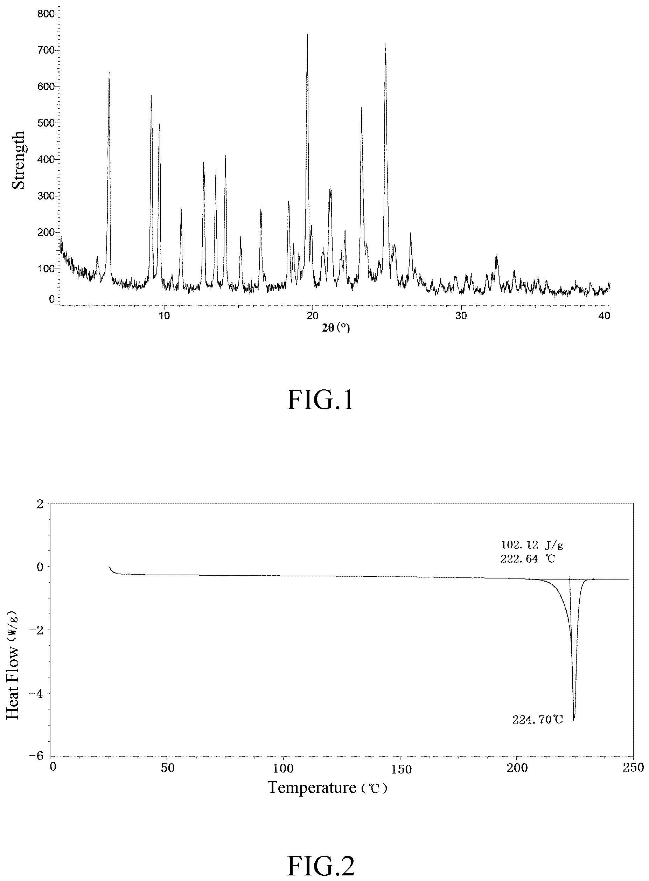

In some embodiments of the present application, the XRPD pattern of the above-mentioned crystalline form A is substantially as shown in FIG. 1.

In some embodiments of the present application, the XRPD pattern analysis data of the above-mentioned crystalline form A is shown in Table 1:

**Table 1. XRPD profile analysis data of crystalline form A**

| Serial Num ber | 2θ angle (°) | Face spacing (Å) | strength | Relative strength (%) | Serial Numb er | 2θ angle (°) | Face spacing (Å) | strength | Relative strength (%) |
|---|---|---|---|---|---|---|---|---|---|
| 1 | 6.305 | 14.00808 | 630.939 | 82.70 | 13 | 19.637 | 4.5172 | 742.661 | 100.00 |
| 2 | 9.140 | 9.66805 | 566.094 | 75.00 | 14 | 19.904 | 4.45707 | 200.832 | 20.70 |
| 3 | 9.681 | 9.12904 | 496.6 | 65.00 | 15 | 20.692 | 4.28916 | 141.575 | 12.00 |
| 4 | 11.138 | 7.9378 | 264.588 | 32.20 | 16 | 21.161 | 4.19514 | 314.742 | 37.30 |
| 5 | 12.661 | 6.98602 | 379.401 | 48.50 | 17 | 21.915 | 4.05255 | 144.651 | 12.50 |
| 6 | 13.479 | 6.56364 | 369.379 | 46.90 | 18 | 22.159 | 4.00839 | 186.116 | 18.60 |
| 7 | 14.139 | 6.25889 | 385.36 | 49.50 | 19 | 23.281 | 3.81764 | 527.583 | 68.20 |
| 8 | 15.175 | 5.83381 | 168.959 | 18.30 | 20 | 23.630 | 3.76214 | 163.475 | 14.70 |
| 9 | 16.523 | 5.36066 | 265.914 | 32.50 | 21 | 24.892 | 3.57412 | 670.036 | 88.80 |
| 10 | 18.385 | 4.82184 | 281.92 | 33.20 | 22 | 25.491 | 3.49145 | 162.618 | 15.00 |
| 11 | 18.710 | 4.7388 | 153.959 | 14.20 | 23 | 26.582 | 3.35058 | 191.692 | 20.40 |
| 12 | 19.094 | 4.64436 | 128.188 | 10.20 | 24 | 32.389 | 2.76196 | 134.152 | 14.10 |

In some embodiments of the present application, the ditterential scanning calorimetry profile of the above-mentioned crystalline form A has an onset value of an endothermic peak at 222.64±3.0°C.

In some embodiments of the present application, the DSC profile of the above-mentioned crystalline form A is shown in FIG. 2.

In some embodiments of the present application, the thermogravimetric analysis profile of the above-mentioned crystalline form A shows a weight loss of 1.220% at 242±3°C.

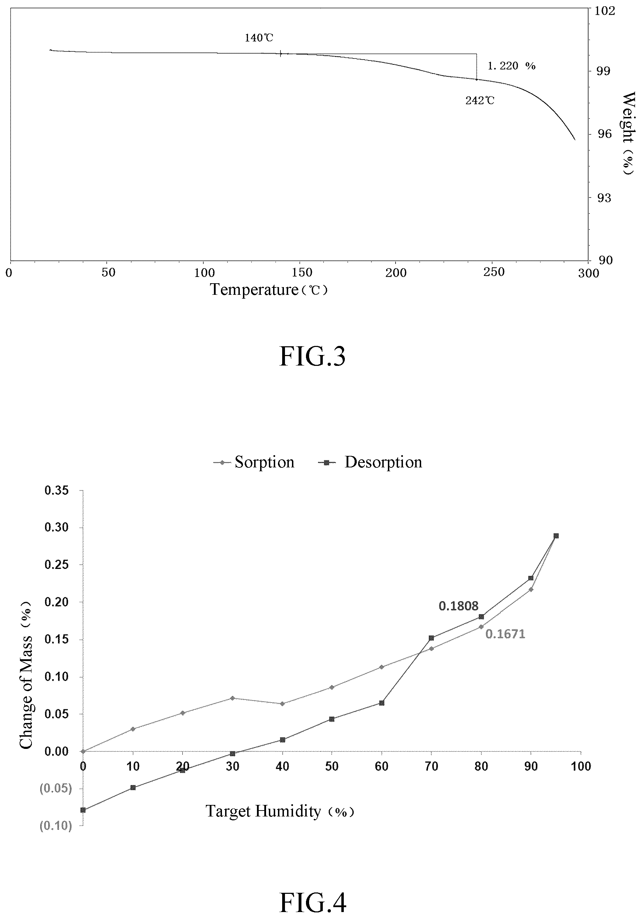

In some embodiments of the present application, the TGA profile of the above-mentioned crystalline form A is shown in FIG. 3.

The present application provides a crystalline form B of a compound of formula (II), wherein m is selected from 0.8, 0.9, 1.0, 1.1 and 1.2, characterized in that its X-ray powder diffraction pattern has characteristic diffraction peaks at the following 2θ angles: 13.855±0.200°, 17.861±0.200°, 19.915±0.200°, and 24.154±0.200°,

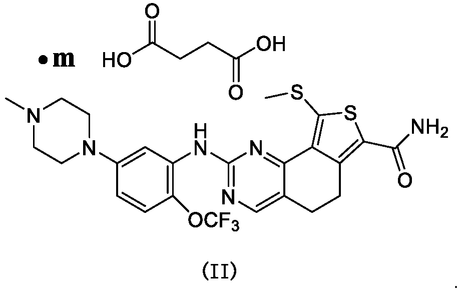

In some embodiments of the present application, the X-ray powder diffraction pattern of the above-mentioned crystalline form B has characteristic diffraction peaks at the following 2θ angles: 13.855±0.200°, 17.861±0.200°, 18.764±0.200°, 19.915±0.200°, 20.651±0.200°, and 24.154±0.200°.

In some embodiments of the present application, the X-ray powder diffraction pattern of the above-mentioned crystalline form B has characteristic diffraction peaks at the following 2θ angles: 7.333±0.200°, 13.855±0.200°, 17.861±0.200°, 18.764±0.200°, 19.915±0.200°, 20.651±0.200°, 24.154±0.200°, and 27.518±0.200°.

In some embodiments of the present application, the X-ray powder diffraction pattern of the above-mentioned crystalline form B has characteristic diffraction peaks at the following 2θ angles: 13.855±0.200°, 17.861±0.200°, 18.764±0.200°, 19.915±0.200°, 20.651±0.200°, 23.449±0.200°, 24.154±0.200°, and 27.518±0.200°.

In some embodiments of the present application, the X-ray powder diffraction pattern of the above-mentioned crystalline form B has characteristic diffraction peaks at the following 2θ angles: 7.333±0.200°, 11.034±0.200°, 13.855±0.200°, 14.350°±0.200°, 16.116±0.200°, 17.861±0.200°, 18.764±0.200°, 19.915±0.200°, 20.651±0.200°, 21.157±0.200°, 24.154±0.200°, and 27.518±0.200°.

In some embodiments of the present application, the X-ray powder diffraction pattern of the above-mentioned crystalline form B has characteristic diffraction peaks at the following 2θ angles: 7.333±0.200°, 11.034±0.200°, 13.855±0.200°, 16.116±0.200°, 17.861±0.200°, 18.764±0.200°, 19.915±0.200°, 20.651±0.200°, 21.157±0.200°, 23.449±0.200°, 24.154±0.200°, and 27.518±0.200°.

In some embodiments of the present application, the X-ray powder diffraction pattern of the above-mentioned crystalline form B has characteristic diffraction peaks at the following 2θ angles: 7.333±0.200°, 11.034±0.200°, 12.635±0.200°, 13.855±0.200°, 14.350±0.200°, 16.116±0.200°, 17.861±0.200°, 18.764±0.200°, 19.915±0.200°, 20.651±0.200°, 21.157±0.200°, 24.154±0.200°, 24.702±0.200°. 0.200°, 26.884±0.200°, 27.518±0.200°, and 31.071±0.200°.

In some embodiments of the present application, the X-ray powder diffraction pattern of the above-mentioned crystalline form B has characteristic diffraction peaks at the following 2θ angles: 7.333±0.200°, 11.034±0.200°, 12.635±0.200°, 13.855±0.200°, 14.350±0.200°, 16.116±0.200°, 17.861±0.200°, 18.764±0.200°, 19.915±0.200°, 20.651±0.200°, 21.157±0.200°, 23.449±0.200°, 24.154±0.200°. 0.200°, 24.702±0.200°, 27.518±0.200°, and 31.071±0.200°.

In some embodiments of the present application, the X-ray powder diffraction pattern of the above-mentioned crystalline form B has characteristic diffraction peaks at the following 2θ angles: 13.855±0.200°, 17.861±0.200°, and/or 7.333±0.200°, and/or 11.034±0.200°, and/or 12.635±0.200°, and/or 13.534±0.200°, and/or 14.350±0.200°, and/or 16.116±0.200°, and/or 17.483±0.200°, and/or 18.764±0.200°, and/or 19.658±0.200°, and/or 19.915±0.200°, and/or 20.651±0.200°, and/or 21.157±0.200°, and/or 23.449±0.200°, and/or 23.806±0.200°, and/or 24.154±0.200°, and/or 24.702±0.200°, and/or 26.884±0.200°, and/or 27.518±0.200°, and/or 31.071±0.200°.

In some embodiments of the present application, the X-ray powder diffraction pattern of the above-mentioned crystalline form B has characteristic diffraction peaks at the following 2θ angles: 7.333°, 11.034°, 13.534°, 13.855°, 14.350°, 16.116°, 17.483°, 17.861°, 18.764°, 19.915°, 20.651°, 21.157°, 23.449°, 23.806°, 24.154°, and 27.518°.

In some embodiments of the present application, the X-ray powder diffraction pattern of the above-mentioned crystalline form B has characteristic diffraction peaks at the following 2θ angles: 7.333°, 11.034°, 12.635°, 13.534°, 13.855°, 14.350°, 16.116°, 17.483°, 17.861°, 18.764°, 19.658°, 19.915°, 20.651°, 21.157°, 23.449°, 23.806°, 24.154°, 24.702°, 26.884°, 27.518°, and 31.071°.

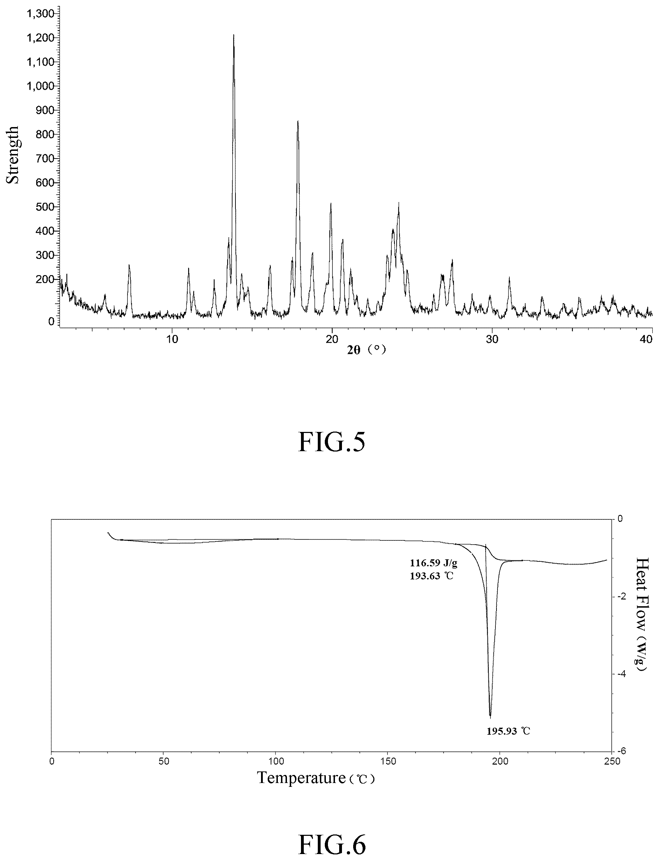

In some embodiments of the present application, the XRPD pattern of the above-mentioned crystalline form B is substantially as shown in FIG. 5.

In some embodiments of the present application, the XRPD profile analysis data of the above-mentioned crystalline form B is shown in Table 2:

**Table 2. XRPD profile analysis data of crystalline form B**

| serial number | 2θ angle (°) | Face spacing (Å) | strength | Relative strength (%) | serial numb er | 2θ angle (°) | Face spacing (Å) | strength | Relative strength (%) |
|---|---|---|---|---|---|---|---|---|---|
| 1 | 7.333 | 12.0457 1 | 254.087 | 18.00 | 12 | 19.915 | 4.45467 | 514.019 | 39.40 |
| 2 | 11.034 | 8.01202 | 247 | 17.40 | 13 | 20.651 | 4.29766 | 355.261 | 25.60 |
| 3 | 12.635 | 7.00008 | 194.722 | 12.40 | 14 | 21.157 | 4.19597 | 239.101 | 15.70 |
| 4 | 13.534 | 6.53722 | 353.402 | 25.90 | 15 | 23.449 | 3.79071 | 294.425 | 19.80 |
| 5 | 13.855 | 6.38647 | 1201.65 | 100.00 | 16 | 23.806 | 3.73473 | 393.244 | 28.30 |
| 6 | 14.350 | 6.16718 | 223.476 | 14.60 | 17 | 24.154 | 3.6817 | 497.458 | 37.20 |
| 7 | 16.116 | 5.49541 | 247.825 | 17.10 | 18 | 24.702 | 3.60124 | 232.601 | 14.10 |
| 8 | 17.483 | 5.06856 | 277.447 | 19.30 | 19 | 26.884 | 3.3137 | 201.019 | 11.80 |
| 9 | 17.861 | 4.96217 | 855.196 | 69.60 | 20 | 27.518 | 3.2388 | 274.531 | 18.50 |
| 10 | 18.764 | 4.72519 | 306.244 | 21.40 | 21 | 31.071 | 2.87602 | 205 | 13.80 |
| 11 | 19.658 | 4.51249 | 182.8 | 10.50 | | | | | |

In some embodiments of the present application, the differential scanning calorimetry profile of the above-mentioned crystalline form B has an onset value of an endothermic peak at 193.63±3.0°C.

In some embodiments of the present application, the DSC profile of the above-mentioned crystalline form B is shown in FIG. 6.

In some embodiments of the present application, the thermogravimetric analysis profile of the above-mentioned crystalline form B shows a weight loss of 0.867% at 92±3°C.

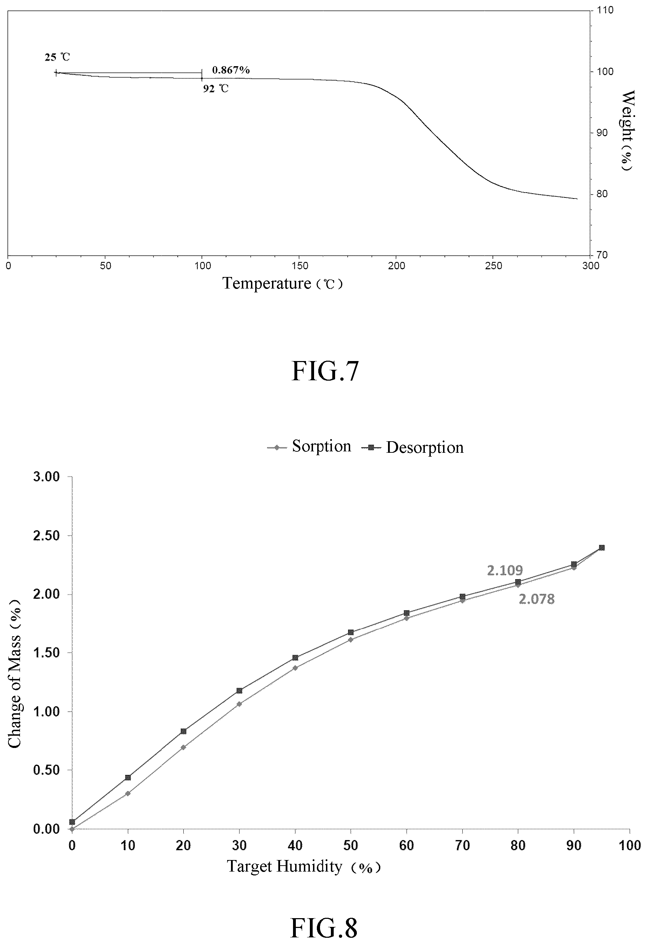

In some embodiments of the present application, the TGA profile of the above-mentioned crystalline form B is shown in FIG. 7.

In some embodiments of the present application, in the above-mentioned crystalline form B of the formula, m is selected from 1.1.

The present application provides a crystalline form C of a compound of formula (III), wherein n is selected from 0.8, 0.9, 1.0, 1.1 and 1.2, characterized in that its X-ray powder diffraction pattern has characteristic diffraction peaks at the following 2θ angles: 13.637±0.200°, 14.138±0.200°, 17.076±0.200°, and 24.866±0.200°,

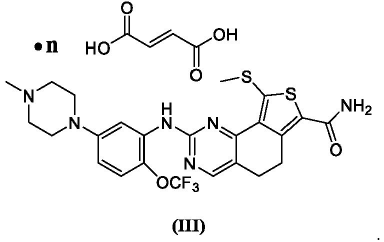

In some embodiments of the present application, the X-ray powder diffraction pattern of the above-mentioned crystalline form C has characteristic diffraction peaks at the following 2θ angles: 11.412±0.200°, 13.637±0.200°, 14.138±0.200°, 17.076±0.200°, 19.310±0.200°, 23.654±0.200°, 24.214±0.200°, and 24.866±0.200°.

In some embodiments of the present application, the X-ray powder diffraction pattern of the above-mentioned crystalline form C has characteristic diffraction peaks at the following 2θ angles: 11.412±0.200°, 13.637±0.200°, 14.138±0.200°, 15.555±0.200°, 17.076±0.200°, 18.016±0.200°, 18.907±0.200°, 19.310±0.200°, 20.811±0.200°, 23.654±0.200°, 24.214±0.200°, and 24.866±0.200°.

In some embodiments of the present application, the X-ray powder diffraction pattern of the above-mentioned crystalline form C has characteristic diffraction peaks at the following 2θ angles: 13.637±0.200°, 17.076±0.200°, 24.866±0.200°, and/or 5.677±0.200°, and/or 7.086±0.200°, and/or 11.412±0.200°, and/or 11.627±0.200°, and/or 13.200±0.200°, and/or 13.429±0.200°, and/or 14.138±0.200°, and/or 14.499±0.200°, and/or 15.167±0.200°. 0.200°, and/or 15.555±0.200°, and/or 15.763±0.200°, and/or 17.272±0.200°, and/or 18.016±0.200°, and/or 18.171±0.200°, and/or 18.614±0.200°, and/or 18.907±0.200°, and/or 19.310±0.200°, and/or 19.656±0.200°, and/or 20.016±0.200°, and/or 20.811±0.200°, and/or 21.765±0.200°, and/or 22.748±0.200°, and/or 23.443±0.200°, and/or 23.654±0.200°, and/or 23.880±0.200°, and/or 24.214±0.200°, and/or 24.577±0.200°, and/or 25.987±0.200°, and/or 26.493±0.200°, and/or 27.011±0.200°, and/or 27.537±0.200°.

In some embodiments of the present application, the X-ray powder diffraction pattern of the above-mentioned crystalline form C has characteristic diffraction peaks at the following 2θ angles: 11.412°, 13.637°, 14.138°, 15.555°, 17.076°, 18.016°, 18.907°, 19.310°, 20.811°, 23.654°, 24.214°, and 24.866°.

In some embodiments of the present application, the X-ray powder diffraction pattern of the above-mentioned crystalline form C has characteristic diffraction peaks at the following 2θ angles: 5.677°, 7.086°, 11.412°, 11.627°, 13.200°, 13.429°, 13.637°, 14.138°, 14.499°, 15.167°, 15.555°, 15.763°, 17.076°, 17.272°, 18.016°, 18.171°, 18.614°, 18.907°, 19.310°, 19.656°, 20.016°, 20.811°, 21.765°, 22.748°, 23.443°, 23.654°, 23.880°, 24.214°, 24.577°, 24.866°, 25.987°, 26.493°, 27.011°, and 27.537°.

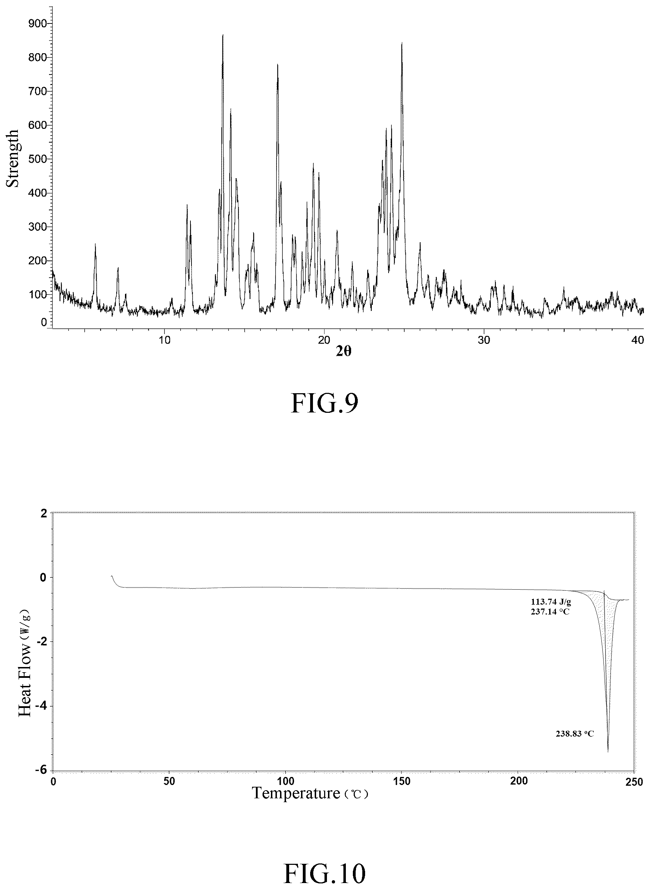

In some embodiments of the present application, the XRPD profile of the above-mentioned crystalline form C is substantially as shown in FIG. 9.

In some embodiments of the present application, the XRPD pattern analysis data of the above-mentioned crystalline form C is shown in Table 3:

**Table 3. XRPD pattern analysis data of crystalline form C**

| serial number | 2θ angle (°) | Face spacing (Å) | strength | Relative strength (%) | serial number | 2θ angle (°) | Face spacing (Å) | strength | Relative strength (%) |
|---|---|---|---|---|---|---|---|---|---|
| 1 | 5.677 | 15.55625 | 246.78 | 23.40 | 18 | 18.907 | 4.68999 | 370.768 | 37.40 |
| 2 | 7.086 | 12.46468 | 174.338 | 16.20 | 19 | 19.310 | 4.59302 | 488 | 52.00 |
| 3 | 11.412 | 7.74789 | 354.173 | 38.00 | 20 | 19.656 | 4.51293 | 457.289 | 48.20 |
| 4 | 11.627 | 7.60458 | 296.091 | 30.70 | 21 | 20.016 | 4.43236 | 202.074 | 16.70 |
| 5 | 13.200 | 6.70204 | 159.051 | 13.10 | 22 | 20.811 | 4.26491 | 288.755 | 27.50 |
| 6 | 13.429 | 6.58837 | 406.704 | 43.70 | 23 | 21.765 | 4.0801 | 191.709 | 15.30 |
| 7 | 13.637 | 6.48789 | 860.956 | 100.00 | 24 | 22.748 | 3.90589 | 150.007 | 10.20 |
| 8 | 14.138 | 6.25919 | 639.07 | 72.20 | 25 | 23.443 | 3.79167 | 348 | 34.30 |
| 9 | 14.499 | 6.10417 | 439.469 | 47.40 | 26 | 23.654 | 3.75836 | 496.437 | 52.60 |
| 10 | 15.167 | 5.837 | 163.508 | 13.30 | 27 | 23.880 | 3.72327 | 586.987 | 63.70 |
| 11 | 15.555 | 5.6923 | 267.268 | 26.40 | 28 | 24.214 | 3.67274 | 593.149 | 64.20 |
| 12 | 15.763 | 5.61759 | 176.241 | 15.30 | 29 | 24.577 | 3.6193 | 297.781 | 27.40 |
| 13 | 17.076 | 5.18848 | 770.817 | 88.50 | 30 | 24.866 | 3.57789 | 840.849 | 94.90 |
| 14 | 17.272 | 5.13003 | 428.015 | 45.60 | 31 | 25.987 | 3.42597 | 237.597 | 20.40 |
| 15 | 18.016 | 4.91967 | 271.351 | 25.40 | 32 | 26.493 | 3.36169 | 158.619 | 11.10 |
| 16 | 18.171 | 4.8782 | 269.562 | 25.00 | 33 | 27.011 | 3.29833 | 149.647 | 10.40 |
| 17 | 18.614 | 4.76297 | 212.955 | 17.80 | 34 | 27.537 | 3.23652 | 148.863 | 10.40 |

In some embodiments of the present application, the differential scanning calorimetry profile of the above-mentioned crystalline form C has an endothermic peak at 237.14±3.0°C.

In some embodiments of the present application, the DSC profile of the above-mentioned crystalline form C is shown in FIG. 10.

In some embodiments of the present application, the thermogravimetric analysis profile of the above-mentioned crystalline form C shows a weight loss of 0.469% at 100±3°C.

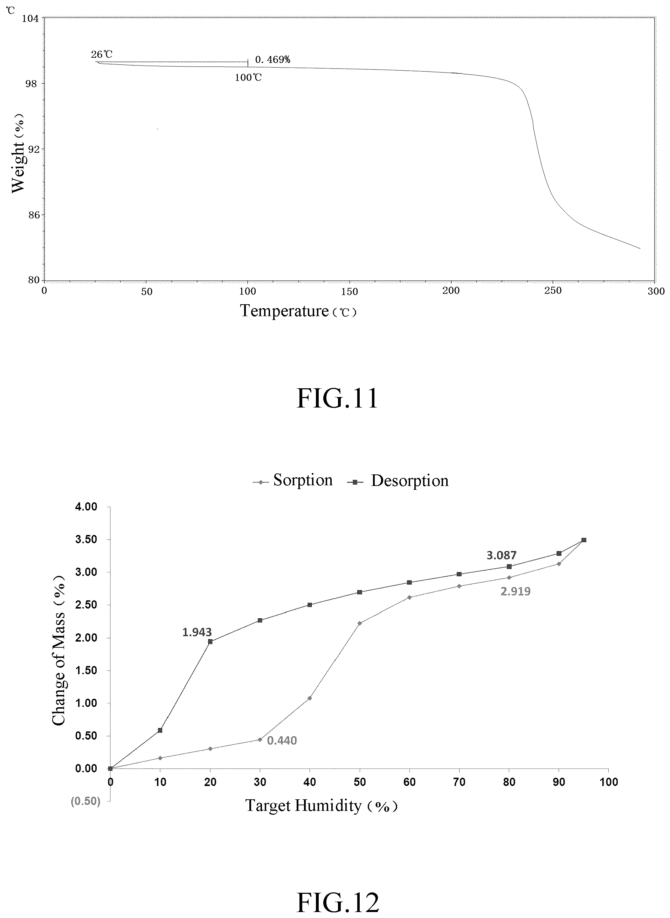

In some embodiments of the present application, the TGA profile of the above-mentioned crystalline form C is shown in FIG. 11.

In some embodiments of the present application, n of the above-mentioned C Crystalline form is selected from 1.0.

The present application provides a crystalline form D of a compound of formula (IV), wherein p is selected from 0.8, 0.9, 1.0, 1.1 and 1.2, characterized in that its X-ray powder diffraction pattern has characteristic diffraction peaks at the following 2θ angles: 6.859±0.200°, 8.784±0.200°, 16.420±0.200°, and 18.965±0.200°,

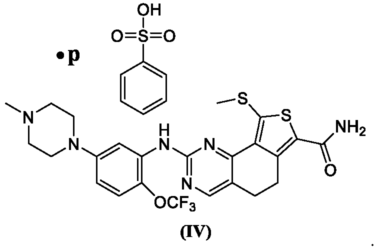

In some embodiments of the present application, the X-ray powder diffraction pattern of the above-mentioned crystalline form D has characteristic diffraction peaks at the following 2θ angles: 6.859±0.200°, 8.784±0.200°, 10.922±0.200°, 13.238±0.200°, 16.420±0.200°, 18.965±0.200°, 20.400±0.200°, and 24.069±0.200°.

In some embodiments of the present application, the X-ray powder diffraction pattern of the above-mentioned crystalline form D has characteristic diffraction peaks at the following 2θ angles: 6.859±0.200°, 8.784±0.200°, 10.922±0.200°, 11.644±0.200°, 13.238±0.200°, 16.420±0.200°, 17.596±0.200°, 18.965±0.200°, 19.390±0.200°, 20.400±0.200°, 21.416±0.200°, and 24.069±0.200°.

In some embodiments of the present application, the X-ray powder diffraction pattern of the above-mentioned crystalline form D has characteristic diffraction peaks at the following 2θ angles: 6.859±0.200°, 8.784±0.200°, and/or 10.922±0.200°, and/or 11.644±0.200°, and/or 13.238±0.200°, and/or 13.741±0.200°, and/or 16.420±0.200°, and/or 16.720±0.200°, and/or 17.596±0.200°, and/or 17.930±0.200°, and/or 18.965±0.200°, and/or 19.390±0.200°. 0.200°, and/or 20.400±0.200°, and/or 21.416±0.200°, and/or 22.630±0.200°, and/or 23.473±0.200°, and/or 24.069±0.200°, and/or 24.832±0.200°, and/or 26.587±0.200°, and/or 27.661±0.200°.

In some embodiments of the present application, the X-ray powder diffraction pattern of the above-mentioned crystalline form D has characteristic diffraction peaks at the following 2θ angles: 6.859°, 8.784°, 10.922°, 11.644°, 13.238°, 16.420°, 17.596°, 17.930°, 18.965°, 19.390°, 20.400°, and 24.069°.

In some embodiments of the present application, the X-ray powder diffraction pattern of the above-mentioned crystalline form D has characteristic diffraction peaks at the following 2θ angles: 6.859°, 8.784°, 10.922°, 11.644°, 13.238°, 13.741°, 16.420°, 16.720°, 17.596°, 17.930°, 18.965°, 19.390°, 20.400°, 21.416°, 22.630°, 23.473°, 24.069°, 24.832°, 26.587°, and 27.661°.

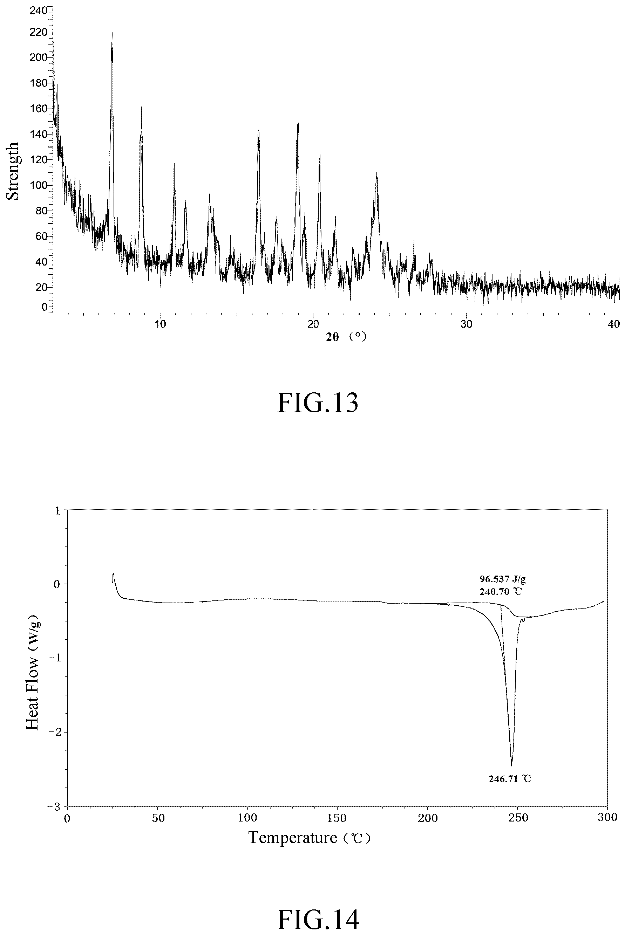

In some embodiments of the present application, the XRPD profile of the above-mentioned crystalline form D is substantially as shown in FIG. 13.

In some embodiments of the present application, the XRPD pattern analysis data of the above-mentioned crystalline form D is shown in Table 4.

**Table 4. XRPD pattern analysis data of crystalline form D**

| serial number | 2θ angle (°) | Face spacing (Å) | strength | Relative strength (%) | serial numb er | 2θ angle (°) | Face spacing (Å) | strength | Relative strength (%) |
|---|---|---|---|---|---|---|---|---|---|
| 1 | 6.859 | 12.87744 | 215.909 | 100.00% | 11 | 18.965 | 4.67575 | 146.51 | 71.90% |
| 2 | 8.784 | 10.05835 | 157.985 | 73.80% | 12 | 19.390 | 4.57403 | 74 | 27.50% |
| 3 | 10.922 | 8.09383 | 106.177 | 43.20% | 13 | 20.400 | 4.34996 | 103.546 | 45.90% |
| 4 | 11.644 | 7.59348 | 82.9602 | 30.00% | 14 | 21.416 | 4.14574 | 53.0737 | 16.20% |
| 5 | 13.238 | 6.68283 | 93.3143 | 37.50% | 15 | 22.630 | 3.92601 | 49.7572 | 15.30% |
| 6 | 13.741 | 6.43932 | 55.2273 | 15.00% | 16 | 23.473 | 3.78695 | 49.8009 | 13.90% |
| 7 | 16.420 | 5.39406 | 128.281 | 60.70% | 17 | 24.069 | 3.69441 | 95.3462 | 41.60% |
| 8 | 16.720 | 5.29809 | 49.0402 | 12.10% | 18 | 24.832 | 3.58262 | 46.4178 | 12.20% |
| 9 | 17.596 | 5.03632 | 74.5938 | 27.60% | 19 | 26.587 | 3.35004 | 45.5258 | 14.20% |
| 10 | 17.930 | 4.94327 | 57.4 | 17.10% | 20 | 27.661 | 3.22235 | 40.7573 | 12.10% |

In some embodiments of the present application, the differential scanning calorimetry profile of the above-mentioned crystalline form D has an onset value of an endothermic peak at 240.70±3.0°C.

In some embodiments of the present application, the DSC profile of the above-mentioned crystalline form D is shown in FIG. 14.

In some embodiments of the present application, the thermogravimetric analysis profile of the above-mentioned crystalline form D shows a weight loss of 0.295% at 120±3°C.

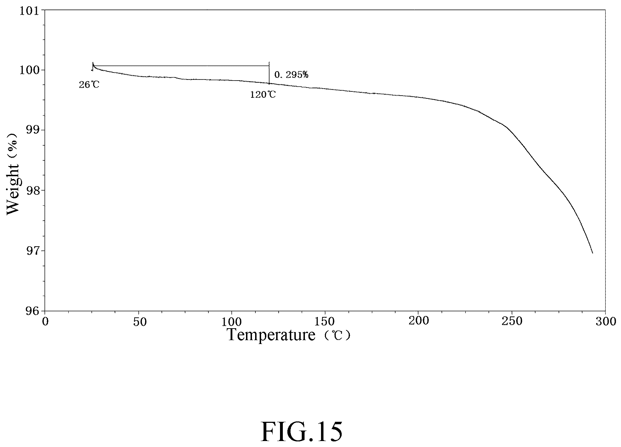

In some embodiments of the present application, the TGA profile of the above-mentioned crystalline form D is shown in FIG. 15.

In some embodiments of the present application, in the above-mentioned crystalline form D of the formula, p is selected from 0.9.

The present application also provides the use of the above-mentioned salt form, the crystalline form A of the compound of formula (I), the crystalline form B of the compound of formula (II), the crystalline form C of the compound of formula (III) and the crystalline form D of the compound of formula (IV) in the preparation of a drug for treating solid tumors.

In some embodiments of the present application, the above-mentioned drug for treating solid tumors is a drug for treating colorectal cancer.

### Technical Effects

The crystalline form of the present application has good stability, good hygroscopicity and good drug development prospects, as well as has good inhibitory effect on PLK1, good pharmacokinetic properties and good oral bioavailability.

### Definition and Description

Unless otherwise specified, the following terms and phrases used herein are intended to have the following meanings. A particular phrase or term should not be considered to be uncertain or unclear in the absence of a special definition, but should be understood according to its ordinary meaning. When a trade name appears in this article, it is intended to refer to its corresponding commercial product or its active ingredient.

The intermediate compounds of the present application can be prepared by a variety of synthetic methods well known to those skilled in the art, including the specific embodiments listed below, embodiments formed by combining them with other chemical synthesis methods, and equivalent substitutions well known to those skilled in the art. Preferred embodiments include but are not limited to the examples of the present application.

The chemical reactions of the specific embodiments of the present application are carried out in a suitable solvent, which must be suitable for the chemical changes of the present application and the reagents and materials required. In order to obtain the compounds of the present application, it is sometimes necessary for those skilled in the art to modify or select the synthesis steps or reaction processes based on the existing embodiments.

The present application will be described in detail below by way of examples, which are not intended to limit the present application in any way.

All solvents used in the present application were commercially available and used without further purification.

The present application uses the following abbreviations: r.t. represents room temperature; THF represents tetrahydrofuran; NMP represents N-methylpyrrolidone; MeSO₃H represents methanesulfonic acid; DME represents ethylene glycol dimethyl ether; DCM represents dichloromethane; Xphos represents 2-dicyclohexylphosphino-2'4'6'-triisopropylbiphenyl; EtOAc represents ethyl acetate; MeOH represents methanol; acetone represents acetone; 2-Me-THF represents 2-methyltetrahydrofuran; and IPA represents isopropanol.

The compounds were named manually or using ChemDraw^{®} software, and commercially available compounds were named according to the supplier's catalog names.

### X-ray powder diffraction (x-ray powder diffractometer XRPD) method of the present application

Instrument model: Bruker D2 PHASER X-ray diffractometer
Test method: Approximately 5 ~ 10 mg of sample was used for XRPD analysis.

The detailed XRPD parameters are as follows:
Light pipe: Cu, kα, (λ= 1.54184Ǻ).
Light tube voltage: 30 kV, light tube current: 10 mA
Divergence slit: 0.60 mm
Detector slit: 0.075 mm
Anti-scatter slit: 0 mm
Scan Range: 3-40 deg
Step size: 0.02 deg
Dwell time per step: 0.2 seconds

### Differential Scanning Calorimeter (DSC) method of the present application

Instrument model: TA Instruments DSC 250 Differential Scanning Calorimeter
Test method: Weigh 1 ~ 3 mg of sample and put it into the sample pan, weigh accurately, record the weight, and test after piercing a hole in an aluminum crucible. The heating rate is from 25 /min ° C to the final temperature.

### Thermogravimetric analysis (TGA) method of the present application

Instrument model: TA Instruments TGA 550 Thermogravimetric Analyzer
Test method: Take 2 ~ 5 mg of sample into the sample pan (Al₂ O₃₎, test in the open air, and heat from 25 ° C to the specified temperature at a heating rate of 10 ° C /min.

### Dynamic Vapor Sorption (DVS) method of the present application

Instrument model: SMS DVS Intrinsic Dynamic Vapor Sorption Instrument
Test conditions: Take a sample (10 ~ 30 mg) and place it in the DVS sample pan for testing.

The detailed DVS parameters are as follows:
Temperature: 25°C
Balance: dm/dt=0.002 %/min (minimum: 10 min, maximum: 180 min)
RH (%) test level: 10% (90%RH-0%RH-90%RH), 5% (95%RH-90%RH and 90%RH-95%RH)
RH (%) test step range: 0%RH-95%RH-0%RH
The classification of hygroscopicity evaluation is shown in Table 5:

**Table 5. Hygroscopicity classification**

| **Hygroscopicity classification** | ΔW% |
|---|---|
| deliquescence | Absorb enough water to form a liquid |
| Highly hygroscopic | ΔW% ≥ 15% |
| Hygroscopic | 15% > ΔW% ≥ 2% |
| Slightly hygroscopic | 2% > ΔW% ≥ 0.2% |
| No or almost no hygroscopicity | ΔW% < 0.2% |

| | |
|---|---|
| Note: ΔW% indicates the weight gain of the test sample at 25±1°C and 80±2% RH. | |

### BRIEF DESCRIPTION OF THE DRAWINGS

FIG.1 is a Cu-Kα radiation XRPD profile of the crystalline form A of the compound of formula (I);
FIG.2 is a DSC profile of the crystalline form A of the compound of formula (I);
FIG.3 is a TGA profile of the crystalline form A of the compound of formula (I);
FIG.4 is a DVS profile of the crystalline form A of the compound of formula (I);
FIG.5 is a Cu-Kα radiation XRPD profile of the crystalline form B of the compound of formula (II);
FIG.6 is a DSC profile of the crystalline form B of compound of formula (II);
FIG.7 is a TGA profile of the crystalline form B of compound of formula (II);
FIG.8 is a DVS profile of the crystalline form B of compound of formula (II);
FIG.9 is a Cu-Kα radiation XRPD profile of the Crystalline form C of the compound of formula (III);
FIG. 10 is a DSC profile of the crystalline form C of the compound of formula (III);
FIG. 11 is a TGA profile of the crystalline form C of the compound of formula (III);
FIG.12 is a DVS profile of the crystalline form C of the compound of formula (III);
FIG. 13 is a Cu-Kα radiation XRPD profile of the crystalline form D of the compound of formula (IV);
FIG.14 is a DSC profile of the crystalline form D of the compound of formula (IV);
FIG. 15 is a TGA profile of the crystalline form D of compound of formula (IV).

### DETAILED DESCRIPTION

The present application is described in detail below by way of examples, but it is not intended to impose any adverse limitations on the present application. The present application has been described in detail herein, and specific embodiments thereof are also disclosed therein. It will be apparent to those skilled in the art that various changes and modifications may be made to the specific embodiments of the present application without departing from the spirit and scope of the present application.

### Example 1: Preparation of the compound of formula (I)

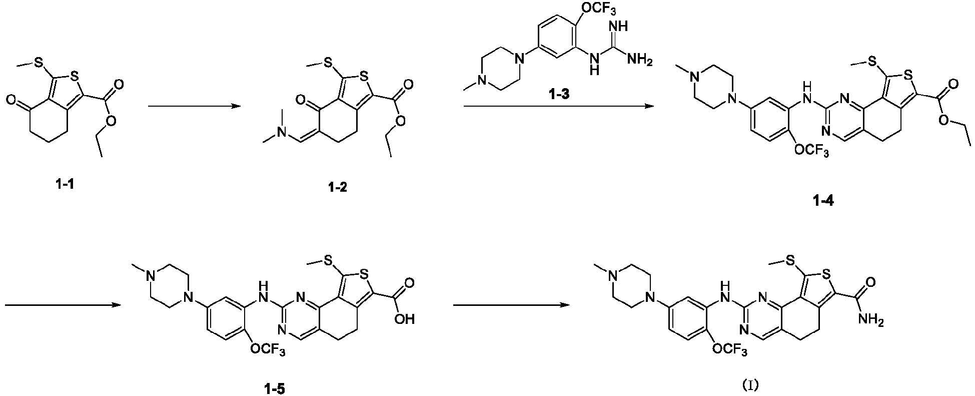

### Step 1: Synthesis of compound 1-2

Compound 1-1 (60 g) was dissolved in tetrahydrofuran (600 mL), and tert-butoxybis(dimethylamino)methane (77.35 g) was added at 20°C. After the addition, the temperature was raised to 80°C for reaction for 12 hours. The temperature was lowered to 20°C, water (200 mL) was added to the reaction solution, and then the solution was concentrated under reduced pressure. Water (400 mL) was added to the reaction solution and stirred for 3 hours. The solution was filtered, the solid was collected, and air-dried to obtain a crude product. 2-Methyltetrahydrofuran (160 mL) and methyl tert-butyl ether (480 mL) were added to the crude product, and the solution was stirred at 20°C for 3 hours. The solution was filtered, the solid was collected, and the solid was dried under reduced pressure to obtain compound **1-2.** LCMS: m/z (ESI) =326.3 [M+H] ^{+. 1} H NMR (400 MHz, CDCl ₃₎ δ: 7.59 (s, 1H), 4.35 - 4.29 (m, 2H), 3.19 - 3.16 (t, 2H, *J* = 6.8 Hz), 3.12 (s, 6H), 2.89 - 2.86 (t, 2H, *J =* 6.8 Hz), 2.58 (s, 3H), 1.39 - 1.35 (t, 3H, *J* =7.0 Hz).

### Step 2: Synthesis of Compound 1-4

Compound **1-3** (78 g) was dissolved in dimethyl sulfoxide (640 mL), and compound **1-2** (80 g) was added. The temperature was raised to 110°C and the mixture was reacted for 48 hours. The mixture was cooled to 30°C, diluted with water (3 L), stirred for 0.5 hours, filtered, rinsed with water (200 mL), and the filter cake was collected. Acetonitrile (750 mL) was added to the filter cake, stirred at 50°C for 2 hours, filtered, and rinsed with acetonitrile (200 mL). Acetonitrile (400 mL) was added to the filter cake, and stirred at 25°C for 16 hours. The mixture was filtered, rinsed with acetonitrile (80 mL), and the filter cake was collected. The filter cake was dried under reduced pressure to obtain compound **1-4.** LCMS: m/z (ESI) =580.2 [M+H] ^{+. 1} H NMR (400 MHz, DMSO- *d ₆₎* δ: 8.55 (s, 1H), 8.34 (s, 1H), 7.49 - 7.46 (m, 1H), 7.19 - 7.17 (m, 1H), 6.73 - 6.71 (m, 1H), 4.31 - 4.25 (m, 2H), 3.32 - 3.15 (m, 6H), 2.79 - 2.76 (m 2H), 2.57 - 2.44 (m 7H), 2.22 (s, 3H), 1.32 - 1.28 (m, 3H).

### Step 3: Synthesis of Compound 1-5

Compound **1-4** (86.8 g) was dissolved in anhydrous tetrahydrofuran (520 mL), and then a solution of lithium hydroxide monohydrate (18.9 g) in water (780 mL) was added to the system, followed by methanol (87 mL), and the temperature was raised to 36.5°C for reaction for 16 hours. The reaction solution was concentrated under reduced pressure to remove the organic solvent to obtain a crude product, and water (780 mL) and 2-methyltetrahydrofuran (78 mL) were added to the crude product. The pH was adjusted to 7 with concentrated hydrochloric acid under stirring, and then adjusted to 6.5 with 1N dilute hydrochloric acid, filtered, and the filter cake was collected. Acetone (800 mL) was added to the filter cake, and the mixture was refluxed for 2 hours under stirring, cooled to 25°C, filtered, and washed with acetone (300 mL). The filter cake was collected and dried under reduced pressure to obtain compound **1-5.** LCMS: m/z (ESI) =552.0 [M+H] ^{+. 1} H NMR (400 MHz, DMSO- *d ₆₎* δ: 8.49 (s, 1H), 8.32 (s, 1H), 7.49 - 7.48 (m, 1H), 6.74 - 6.72 (m, 1H), 6.72 - 6.70 (m, 1H), 3.32 - 3.17 (m, 6H), 2.77 - 2.75 (m, 2H), 2.55 - 2.50 (m, 7H), 2.27 (s, 3H).

### Step 4: Synthesis of compound of formula (I)

To compound 1-5 (10 g), add 2-(7-azobenzotriazole)-N,N,N,N-tetramethyluronium hexafluorophosphate (13.79 g), diisopropylethylamine (7.03 g), and ammonium bicarbonate (4.3 g) in sequence, and stir at 30°C for 16 hours. Slowly pour the reaction solution into water (0.5 L), stirred at 25°C for 16 hours, filtered, collected the filter cake to obtain a crude product, added water (100 mL) to the crude product, stirred at 25°C for 16 hours, filtered, collected the filter cake and dried the residual solvent with a nitrogen stream to obtain the compound of formula (I). LCMS: m/z (ESI) =551.2 [M+H] ^{+. 1} H NMR (400 MHz, DMSO- *d ₆₎* δ:8.45 (s, 1H), 8.32 (s, 1H), 7.52 - 7.51 (m, 1H), 7.34 (s, 2H), 7.18 - 7.15 (m, 1H), 6.72 - 6.68 (m, 1H), 3.17 - 3.10 (m, 6H), 2.75 - 2.71 (m, 2H), 2.54 - 2.46 (m, 7H), 2.23 (s, 3H).

### Example 2: Preparation of the crystalline form A of the compound of formula (I)

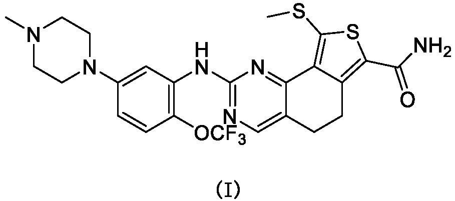

200 mg of the compound of formula (I) was weighed and added to a glass vial containing 3 mL of ethanol to form a suspension. After adding a magnet, the suspension was placed on a magnetic heating stirrer, stirred at 60°C overnight, then the temperature was lowered to room temperature, filtered, and thus-obtained solid was concentrated under reduced pressure using a vacuum pump to render crystalline form A of the compound of formula (I). The XRPD profile is shown in FIG. 1, the DSC profile is shown in FIG. 2, and the TGA profile is shown in FIG.3.

Weigh 200 mg of the compound of formula (I) and add it to a glass vial containing 3 mL of acetone to form a suspension. After adding a magnet, place the suspension on a magnetic heating stirrer, stir at 60°C overnight, then lower the temperature to room temperature, filter, and thus-obtained solid was concentrated under reduced pressure using a vacuum pump to render crystalline form A of the compound of formula (I).

Weigh 200 mg of the compound of formula (I) and add it to a glass vial containing 3 mL of methanol to form a suspension. After adding a magnet, place the suspension on a magnetic heating stirrer, stir at 60°C overnight, then lower the temperature to room temperature, filter, and concentrate obtained solid under reduced pressure using a vacuum pump to render crystalline form A of the compound of formula (I).

Weigh 200 mg of the compound of formula (I) and add it to a glass vial containing 1.5 mL of ethanol and 1.5 mL of water to form a suspension. After adding a magnet, place the suspension on a magnetic heating stirrer, stir at 60°C overnight, then lower the temperature to room temperature, filter, and concentrate obtained solid under reduced pressure using a vacuum pump to render crystalline form A of the compound of formula (I).

Weigh 200 mg of the compound of formula (I) and add it to a glass vial containing 1.5 mL of acetone and 1.5 mL of water to form a suspension. After adding a magnet, place the suspension on a magnetic heating stirrer, stir at 60°C overnight, then lower the temperature to room temperature, filter, and concentrate obtained solid under reduced pressure using a vacuum pump to render crystalline form A of the compound of formula (I).

Weigh 200 mg of the compound of formula (I) and add it to a glass vial containing 3 mL of ethyl acetate to form a suspension. After adding a magnet, place the suspension on a magnetic heating stirrer, stir at 60°C overnight, then lower the temperature to room temperature, filter, and concentrate obtained solid under reduced pressure using a vacuum pump to render crystalline form A of the compound of formula (I). ¹ H NMR (400 MHz, D₂O) δ: 7.90 (s, 1 H), 7.30 (d, *J =* 8.8 Hz, 1 H), 7.14 (d, *J* = 3.2 Hz, 1 H), 7.00 (dd, *J* = 8.8, 3.2 Hz, 1 H), 3.74 -3.77 (m, 2 H), 3.47 - 3.50 (m, 2 H), 2.99 - 3.15 (m, 4 H), 2.74 - 2.83 (m, 5 H), 2.51 - 2.59 (m, 2 H), 2.07 (s, 3 H).

### Example 3: Preparation of Crystalline Form B of Compound (II)

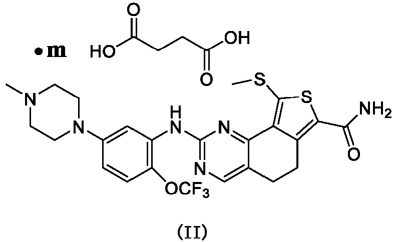

About 50 mg of the crystalline form A of the compound of formula (I) was weighed and added into 1.0 mL of ethanol and stirred at 50°C. 11.38 mg of succinic acid was weighed and diluted with 1 mL of ethanol solvent, and the diluted succinic acid solution was slowly added dropwise to the above crystalline form A solution, stirred at 50°C for 20 h, then cooled to 25°C and stirred for 4 days, filtered and dried at 50°C for about 18 h to obtain the crystalline form B of the compound of formula (II). The XRPD profile is shown in FIG.5, the DSC profile is shown in FIG.6, and the TGA profile is shown in FIG.7.

Weigh 45 mg of succinic acid and add it to a glass vial containing 3 mL of ethanol to dissolve it. Weigh 200 mg of the compound of formula (I) and add it to the above solution. Place the reaction solution on a magnetic heating stirrer, stir at 60°C overnight, then lower the temperature to room temperature, filter, and concentrate the solid under reduced pressure using a vacuum pump to obtain crystal form B of the compound of formula (II).

Weigh 45 mg of succinic acid and add it to a glass vial containing 3 mL of methanol to dissolve it. Weigh 200 mg of the compound of formula (I) and add it to the above solution. Place the reaction solution on a magnetic heating stirrer, stir at 60°C overnight, then lower the temperature to room temperature, filter, and concentrate the solid under reduced pressure using a vacuum pump to obtain crystal form B of the compound of formula (II).

Weigh 45 mg of succinic acid and add it to a glass vial containing 1.5 mL of methanol and 1.5 mL of water to dissolve it. Weigh 200 mg of the compound of formula (I) and add it to the above solution. Place the reaction solution on a magnetic heating stirrer, stir at 60°C overnight, then lower the temperature to room temperature, filter, and concentrate the solid under reduced pressure using a vacuum pump to obtain crystal form B of the compound of formula (II).

Weigh 45 mg of succinic acid and add it to a glass vial containing 1.5 mL of ethanol and 1.5 mL of water to dissolve it. Weigh 200 mg of the compound of formula (I) and add it to the above solution. Place the reaction solution on a magnetic heating stirrer, stir at 60°C overnight, then lower the temperature to room temperature, filter, and concentrate the solid under reduced pressure using a vacuum pump to obtain crystal form B of the compound of formula (II).

Weigh 45 mg of succinic acid and add it to a glass vial containing 3 mL of acetone to dissolve it. Weigh 200 mg of the compound of formula (I) and add it to the above solution. Place the reaction solution on a magnetic heating stirrer, stir overnight at 60°C, then lower the temperature to room temperature, filter, and concentrate the solid under reduced pressure with a vacuum pump to obtain crystal form B of the compound of formula (II). According to the nuclear magnetic spectrum, m is 1.1. ¹ H NMR (400 MHz, D ₂ O) δ: 7.95 (s, 1 H), 7.35 (d, *J* = 9.2 Hz, 1 H), 7.18 (d, *J* = 2.4 Hz, 1 H), 7.04 (dd, *J* = 9.2, 2.4 Hz, 1 H), 3.80 (m, 2 H), 3.53 (m, 2 H), 3.01 - 3.20 (m, 4 H), 2.81 - 2.90 (m, 5 H), 2.58 - 2.67 (m, 2 H), 2.53 (s, 4.4 H), 2.18 (s, 3 H).

### Example 4: Preparation of Crystalline Form C of Compound (III)

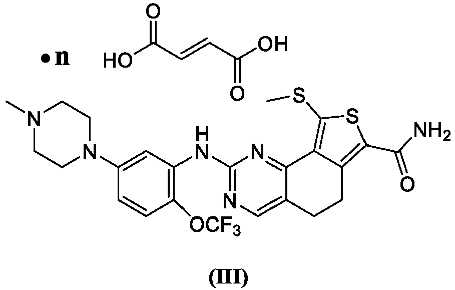

Weigh 155 mg of the crude product in step 4 of Example 1, add water (1.5 mL), add concentrated hydrochloric acid (1.5 mL) to dissolve the crude product, add sodium hydroxide solid to adjust the pH to greater than 14, filter, collect the filter cake, add water (1 mL), stir at 25°C for 6 hours, filter, collect the filter cake, and bake in an oven at 50°C for 16 hours to obtain 150 mg of solid which was added together with 33.2 mg of fumaric acid to a 4.0 mL glass vial, and add 2 mL of ethyl acetate to make it a suspension. Add a magnet into the above suspension and place it on a magnetic heating stirrer. The mixture was stirred at 50°C overnight and then cooled to 25°C with stirring, and then the mixture was filtered and the filter cake was collected to obtain the crystalline form C of the compound of formula (III).

Weigh 520 mg of the crude product in step 4 of Example 1, add water (5.2 mL), add concentrated hydrochloric acid (5.2 mL) to dissolve the crude product, add solid sodium hydroxide to adjust the pH to greater than 14, filter, collect the filter cake, add water (3.7 mL) thereto, stir at 25°C for 6 hours, filter, collect the filter cake, and bake in an oven at 50°C for 16 hours to obtain 500 mg of a solid, which was added to a 40.0 mL glass vial, 5 mL of a mixed solution of dichloromethane and methanol (dichloromethane: methanol = 2:1) was added, a magnet was added and stirred at 50°C to dissolve it, 110.7 mg of fumaric acid was dissolved in 10 mL of a mixed solution of dichloromethane and methanol (dichloromethane: methanol = 2:1), and then added dropwise to the glass vial, the above reaction solution was placed on a magnetic heating stirrer (50°C), stirred at 50°C for 3 hours, and then cooled to 20°C under stirring. After that, filtered, and the filter cake was collected to obtain the crystalline form C of compound of formula (III). According to the analysis of the nuclear magnetic resonance spectrum, n was 1.0. The XRPD profile was shown in FIG. 9, the DS profile was shown in FIG. 10, and the TGA profile was shown in FIG. 11. ¹ H NMR (400 MHz, DMSO- *d ₆₎* δ: 8.48 (s, 1 H), 8.32 (s, 1 H), 7.55 - 7.50 (m, 1 H), 7.44 (s, 2 H), 7.22 - 7.15 (m, 1 H), 6.76 - 6.69 (m, 1 H), 6.60 (s, 2 H), 3.23 - 3.16 (m, 4 H), 3.15 - 3.08 (m, 2 H), 2.79 - 2.70 (m, 2 H), 2.60 - 2.55 (m, 4 H), 2.54 (s, 3 H),2.30 (s, 3 H).

### Example 5: Preparation of Crystalline Form D of Compound (IV)

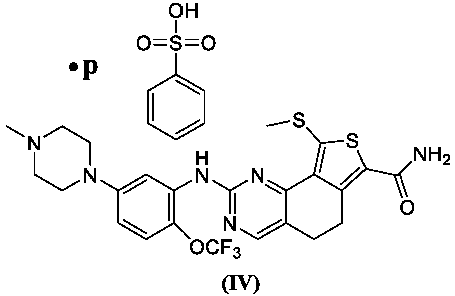

About 50 mg of the crystalline form A of the compound of formula (I) was weighed and added into 1.0 mL of ethanol, and stirred at 50°C. 15.28 mg of benzenesulfonic acid was diluted with 1 mL of ethanol solvent, and the diluted benzenesulfonic acid solution was slowly added dropwise to the above form A ethanol solution, stirred at 50°C for 20h, then cooled to 25°C and stirred for 4 days, filtered and dried at 50°C for about 18 h to obtain crystalline form D of compound (IV). According to the nuclear magnetic spectrum, p was 0.9, the XRPD profile was shown in FIG. 13, the DSC profile was shown in FIG. 14, and the TGA profile was shown in FIG. 15. ¹ H NMR (400 MHz, DMSO- *d ₆₎* δ : 9.37 - 9.69 (m, 0.9 H), 8.59 (s, 1 H), 8.33 (s, 1 H), 7.56 - 7.65 (m, 1.8 H), 7.54 (d, *J* = 3.2 Hz, 1 H), 7.44 - 7. 56 (m, 2 H), 7.27 - 7.36 (m, 2.7 H), 7.24 (d, *J =* 8.4 Hz, 1 H), 6.80 (dd, *J =* 8.4, 3.2 Hz, 1 H), 3.40 - 3.96 (m, 4 H), 2.92 - 3.26 (m, 6 H), 2.86 (s, 3 H), 2.74 (m, 2 H), 2.54 (s, 3 H).

### Example 6: Crystalline form screening experiment

### 6.1 Suspension crystallization

The operation process of suspension crystallization was to weigh a certain amount of the crystalline form A of the compound of formula (I), add a certain amount of solvent, maintain the system in a state of suspension and stir at a certain temperature, and filter out the solid for analysis after a period of time. The specific conditions are shown in Table 6.

**Table 6. Suspension crystallization**

| **Condition** | **Solute/mg** | **Solvents** | **Volume /mL** | **Temperature/°C** | **Result** |
|---|---|---|---|---|---|
| 1 | 40.90 | Acetonitrile | 2.0 | 50 | crystal form A |
| 2 | 40.12 | Ethyl acetate | 2.0 | 50 | crystal form A |
| 3 | 40.65 | Toluene | 2.0 | 50 | crystal form A |
| 4 | 39.19 | Butanone | 1.0 | 50 | crystal form A |

### 6.2 Cooling crystallization

The cooling crystallization operation process was to dissolve a certain amount of the crystalline form A of the compound of formula (I) in a certain amount of solvent at high temperature, stir at high temperature for a period of time, and then cool to a lower temperature at a certain cooling rate. If solid precipitates after cooling to a low temperature, filter it out for solid state analysis. The specific conditions are shown in Table 7.

**Table 7. Cooling crystallization**

| **Condition** | **Solute/mg** | **Solvents** | **Volume /mL** | **Temperature/°C** | **Result** |
|---|---|---|---|---|---|
| 1 | 40.85 | Tetrahydrofuran | 1.0 mL | 50-25 | crystal form A |
| 2 | 40.92 | 2- methyl - tetrahydrofuran | 5.0 mL | 50-25 | crystal form A |

### 6.3 Anti-solvent crystallization

The operation process of anti-solvent crystallization was to dissolve a certain amount of the crystalline form A of the compound of formula (I) in a positive solvent to obtain a solution of the raw material drug, slowly add the raw material drug solution to the anti-solvent at a certain temperature, and filter out any solid precipitation for analysis. The specific conditions are shown in Table 8.

**Table 8. Anti-solvent crystallization**

| **Condition** | **Solute /mg** | **Solvent/ mL** | **Antisolvent/ mL** | **Volume /mL** | **Temperature/°C** | **Result** |
|---|---|---|---|---|---|---|
| 1 | 40.74 | THF | H₂O | 2.0+4.0 | 25 - 5 | crystal form A |
| 2 | 40.73 | THF | IPA | 2.0+6 0. | 25 - 5 | crystal form A |
| 3 | 39.92 | THF | Tol | 2.0+4.0 | 25 - 5 | crystal form A |
| 4 | 40.59 | DCM | ACN | 4.0 | 25 - 5 | crystal form A |
| 5 | 39.73 | DCM | MTBE | 4.0 | 25 - 5 | crystal form A |
| 6 | 40.07 | DMSO | EtOAc | 0.6+8.0 | 25 - 5 | crystal form A |

Experimental conclusion: The crystalline form A of compound of formula (I) has good stability in different solvents and at different temperatures.

### Example 7: Study on Hygroscopicity of Crystalline Form A of Compound of Formula (I)

### Experimental Materials:

SMS DVS Intrinsic Dynamic Vapor Sorption Instrument

### Experimental methods:

10-30 mg of the crystalline form A of the compound of formula (I) was placed in a DVS sample tray for testing.

### Experimental results:

DVS profile of the crystalline form A of the compound of formula (I) is shown in FIG.4. When the humidity rises to 80 %, ΔW = 0.1671%.

### Experimental conclusion:

The crystalline form A of the compound of formula (I) increases by 0.1671% in weight at 25°C and 80% RH, and therefore, the crystalline form A is not hygroscopic.

### Example 8: Study on the Hygroscopicity of Crystalline form B of Compound (II)

### Experimental Materials:

SMS DVS Intrinsic Dynamic Vapor Sorption Instrument

### Experimental methods:

10-30 mg of the crystalline form B of the compound of formula (II) was placed in a DVS sample tray for testing.

### Experimental results:

DVS profile of the crystalline form B of compound (II) is shown in FIG.8. When the humidity rises to 80 %, ΔW = 2.078%.

### Experimental conclusion:

The crystalline form B of the compound of formula (II) increases by 2.078% in weight at 25°C and 80% RH, and therefore, the crystalline form B is hygroscopic.

### Example 9: Study on the Hygroscopicity of Crystalline Form C of Compound (III)

### Experimental Materials:

SMS DVS Intrinsic Dynamic Vapor Sorption Analyzer

### Experimental methods:

10-30 mg of the crystalline form C of the compound of formula (III) was placed in a DVS sample tray for testing.

### Experimental results:

DVS profile of the crystalline form C of the compound of formula (III) is shown in FIG.12. When the humidity rises to 80 %, ΔW = 2.919%.

### Experimental conclusion:

The crystalline form C of the compound of formula (III) increases by 2.919% in weight at 25°C and 80% RH, and therefore, the crystalline form C is hygroscopic.

### Example 10: Solid Stability Test of Crystalline Form A of Formula (I) and Crystalline Form B of Formula (II)

### Experimental Purpose:

According to the "Guidelines for Stability Testing of APIs and Preparations" (Chinese Pharmacopoeia 2020 Edition, Part IV, General Rules 9001), the stabiilty of the crystalline form A of compound of formula (I) and the crystalline form B of compound of formula (II) was investigated at high temperature and high humidity (40°C/75%RH, open) (60°C/75%RH, open) conditions.

### Experimental operation:

Weigh 10-30 mg of the crystalline form A of the formula (I) and the crystalline form B of the compound of formula (II), place them at the bottom of a glass sample bottle, and spread them into a thin layer. The samples were placed open to ensure that the samples were fully in contact with the ambient air. The samples placed under different conditions were sampled and tested (XRPD) on the 10th day, 30th day, and 60th day, and the test results were compared with the initial test results on day 0. The test results are shown in Table 9:

**Table 9. Stability test results of the crystalline form of compound A of formula (I) and the crystalline form B of compound of formula (II)**

| | Test conditions | Time | Crystal form |
|---|---|---|---|
| Crystalline Form A of Compound of Formula (I) | Initial sample | Day 0 | crystal form A |
| | 40 °C /75%RH | 10 days | crystal form A |
| | 60 °C /75%RH | 10 days | crystal form A |
| | 40 °C /75%RH | 30 days | crystal form A |
| | 60 °C /75%RH | 30 days | crystal form A |
| | 40 °C /75%RH | 60 days | crystal form A |
| | 60 °C /75%RH | 60 days | crystal form A |
| Crystalline Form B of Compound of Formula (II) | Initial sample | Day 0 | crystal form B |
| | 40 °C /75%RH | 10 days | crystal form B |
| | 60 °C /75%RH | 10 days | crystal form B |

**Experimental conclusion:** The crystalline form A of compound of formula (I) and the crystalline form B of compound of formula (II) both have good stability under high temperature and high humidity conditions.

### Test Example 1 : Pharmacokinetic study of oral administration of the test compound in male beagle dogs Experimental Purpose:

This study was designed to investigate the pharmacokinetics of the test compound in the plasma of male beagle dogs after oral administration.

### Experimental operation:

Oral administration group: Weigh an appropriate amount of the test compound (calculated in free form), dissolve it in a 0.5% MC aqueous solution, vortex and sonicate for 10 min until the compound was milky white and uniformly suspended, and prepare a 1.0 mg/mL uniform suspension solution for use. Male beagle dogs weighing about 11 kg were selected and orally administered with the test compound. Sample collection time was: 0.083, 0.25, 0.5, 1, 2, 4, 6, 8, and 24 hours.

1 mL of whole blood was collected from the forelimb vein to prepare plasma for concentration determination by high performance liquid chromatography - tandem mass spectrometry (LC-MS/MS). The non-compartmental model of WinNonlin 8.2.0 (Pharsight, Mountain View, CA), pharmacokinetic software, was used to process plasma concentrations, and the linear logarithmic trapezoidal method was used to calculate pharmacokinetic parameters. The experimental results are shown in Table 10:

**Table 10. Pharmacokinetic study results of crystalline form A, crystalline form B and crystalline form C**

| | | Pharmacokinetic study of the test compound after oral administration in male beagle dogs | | |
|---|---|---|---|---|
| PO | Crystalline forms | Crystalline Form A of compound of Formula (I) | Crystalline Form B of compound of Formula (II) | Crystalline Form C of compound of Formula (III) |
| | **Dose (mpk) (Calculated in free state)** | 5.0 | 5.0 | 5.0 |
| | **Cmax (nM)** | 106 | 123 | 132 |
| | **Tmax (h)** | 3.0 | 4.0 | 6.0 |
| | **T _{1/2} (h)** | 4.6 | 5.0 | 4.0 |
| | **AUC ₀₋ₗₐₛₜ (nM.h)** | 907 | 1107 | 1591 |

**Experimental conclusion:** The crystalline form A of compound of formula (I), the crystalline form B of compound of formula (II) and the crystalline form C of compound of formula (III) are all well absorbed in male beagle dogs after oral administration.

### Test Example 2: Evaluation of PLK1 kinase activity in vitro

The IC₅₀ value was determined using ³³P isotope-labeled kinase activity assay (Reaction Biology Corp) to evaluate the inhibitory ability of the test compounds on human PLK1 protein kinase.

Buffer conditions: 20 mM HEPES (pH 7.5), 10 mM MgCl₂, 1 mM EGTA, 0.01% Brij35, 0.02 mg/ml BSA, 0.1 mM Na₃VO_{4,} 2 mM DTT, 1% DMSO

Test steps: Dissolve the test compound in DMSO at room temperature to prepare a 10 mM solution for use. Dissolve the substrate Casein in a freshly prepared buffer (final concentration 20 µM), add the tested PLK1 kinase (final concentration 12 nM) and mix well. Add the DMSO-dissolved test compound stock solution to the above mixed reaction solution using the acoustic pipetting system Echo 550 according to the set final concentration gradient (highest final concentration was 1 µM, 3-fold dilution, 10 gradients). After incubation at room temperature for 20 minutes, add ³³ P-ATP (final concentration 0.01 µCi/µL), incubate at room temperature for 120 minutes, and then spot the reaction solution on P81 ion exchange filter paper (Whatman # 3698-915). After repeatedly washing the filter paper with 0.75% phosphoric acid solution, the level of residual radioactive phosphorylated substrate on the filter paper was determined. % kinase activity = kinase activity test compound/ kinase activity blank group (DMSO) × 100%. The IC₅₀ value was obtained by profile fitting using Prism4 software (GraphPad). The experimental results are shown in Table 11.

**Table 11. Results of in vitro PLK1 kinase activity of the compound of the present application**

| **Compound No.** | **PLK1/IC₅₀ (nM)** |
|---|---|
| **Crystalline Form A of compound of Formula (I)** | 2.48 |

**Experimental conclusion:** Crystalline form A of compound of formula (I) exhibits good inhibitory activity against PLK1.

## Claims

1. Succinate, fumarate, benzenesulfonate, sulfate, phosphate, maleate, L-tartrate, methanesulfonate, L-malate, hydrochloride, citrate or L-aspartate of the compound of formula (I):

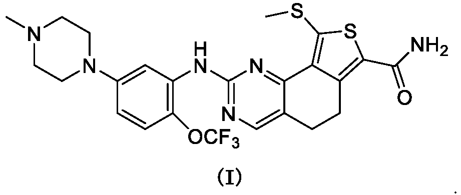

2. The salt of the compound according to claim 1, which is selected from: 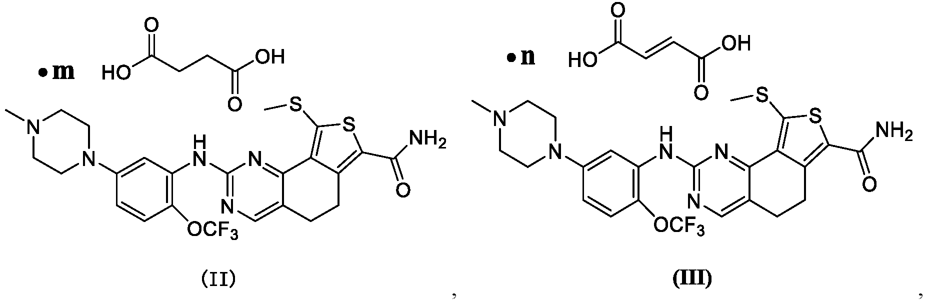 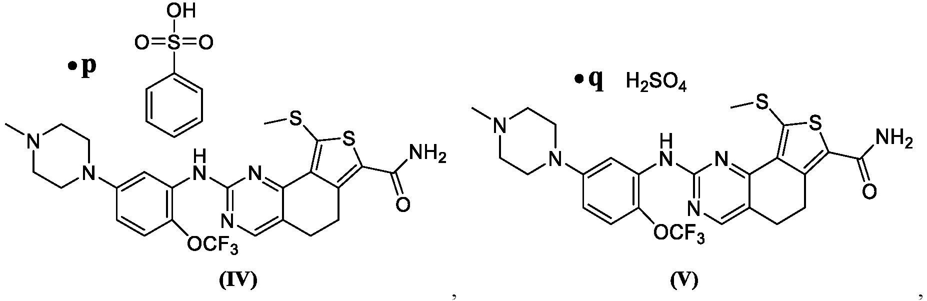 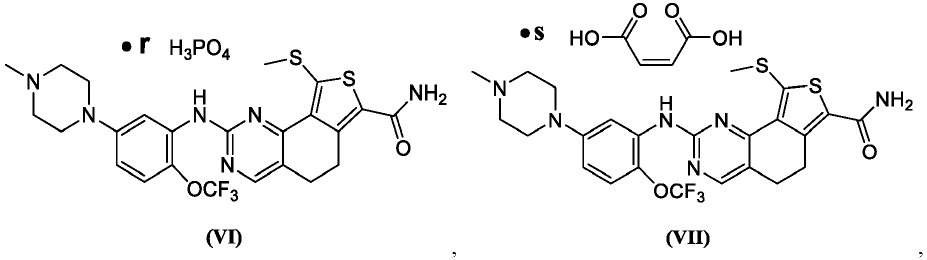 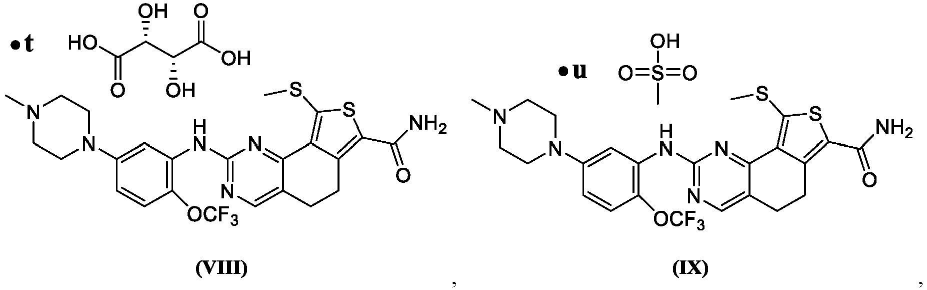 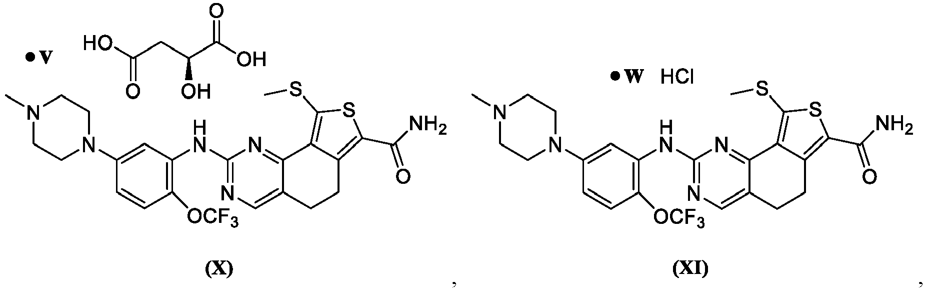 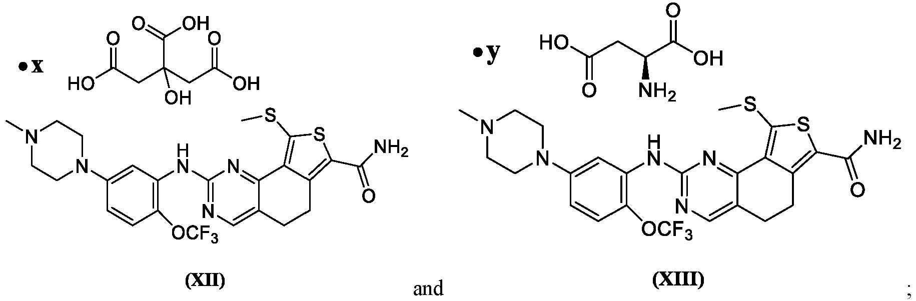 wherein m, n, p, q, r, s, t, u, v, w, x and y are independently selected from 0.8, 0.9, 1.0, 1.1, 1.2, 1.3, 1.4, 1.5, 1.6, 1.7, 1.8, 1.9 and 2.0, respectively.

3. The salt of the compound according to claim 2, wherein m, n, p, q, r, s, t, u, v, w, x and y are each independently selected from 1.0.

4. Crystalline form A of the compound of formula (I), wherein the X-ray powder diffraction pattern comprises characteristic peaks at 2θ angles of 6.305±0.200°, 9.140±0.200°, 12.661±0.200°, and 19.637±0.200°

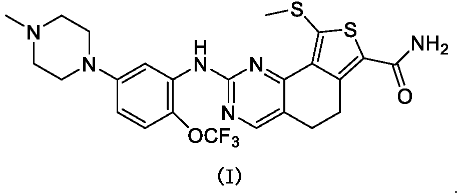

5. The crystalline form A according to claim 4, wherein the X-ray powder diffraction pattern comprises characteristic peaks at 2θ angles of 6.305±0.200°, 9.140±0.200°, 9.681±0.200°, 12.661±0.200°, 13.479±0.200°, 19.637±0.200°, 23.281±0.200°, and 24.892±0.200°.

6. The crystalline form A according to claim 5, wherein the X-ray powder diffraction pattern comprises characteristic peaks at 2θ angles of 6.305±0.200°, 9.140±0.200°, 9.681±0.200°, 12.661±0.200°, 13.479±0.200°, 14.139±0.200°, 16.523±0.200°, 18.385±0.200°, 19.637±0.200°, 21.161±0.200°, 23.281±0.200°, and 24.892±0.200°.

7. The crystalline form A according to claim 6, wherein the X-ray powder diffraction pattern comprises characteristic peaks at 2θ angles of 6.305°, 9.140°, 9.681°, 11.138°, 12.661°, 13.479°, 14.139°, 15.175°, 16.523°, 18.385°, 18.710°, 19.094°, 19.637°, 19.904°, 20.692°, 21.161°, 21.915°, 22.159°, 23.281°, 23.630°, 24.892°, 25.491°, 26.582°, and 32.389°.

8. The crystalline form A according to any of claims 4 to 7, wherein the XRPD pattern is substantially as shown in FIG. 1.

9. The crystalline form A according to any of claims 4 to 7, wherein the differential scanning calorimetry profile has an onset value of an endothermic peak at 222.64±3.0°C.

10. The crystalline form A according to claim 9, wherein the DSC profile is as shown in FIG. 2.

11. The crystalline form A according to any of claims 4 to 7, the thermogravimetric analysis profile of the crystalline form A shows a weight loss of 1.220% at 242±3°C.

12. The crystalline form A according to claim 11, wherein the TGA profile is as shown in FIG. 3.

13. Crystalline form B of the compound of formula (II), wherein m is selected from the group consisitng of 0.8, 0.9, 1.0, 1.1 and 1.2, and the crystalline form B has an X-ray powder diffraction pattern comprising characteristic peaks at 2θ angles of 13.855±0.200°, 17.861±0.200°, 19.915±0.200°, and 24.154±0.200°

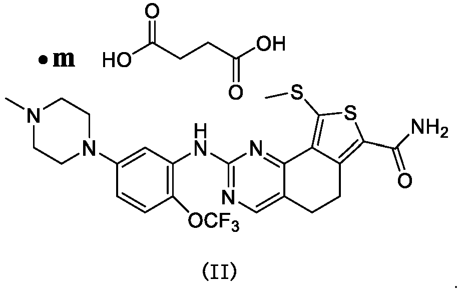

14. The crystalline form B according to claim 13, wherein the X-ray powder diffraction pattern comprises characteristic peaks at 2θ angles of 7.333±0.200°, 13.855±0.200°, 17.861±0.200°, 18.764±0.200°, 19.915±0.200°, 20.651±0.200°, 24.154±0.200°, and 27.518±0.200°.

15. The crystalline form B according to claim 14, wherein the X-ray powder diffraction pattern comprises characteristic peaks at 2θ angles of 7.333±0.200°, 11.034±0.200°, 13.855±0.200°, 14.350°±0.200°, 16.116±0.200°, 17.861±0.200°, 18.764±0.200°, 19.915±0.200°, 20.651±0.200°, 21.157±0.200°, 24.154±0.200°, and 27.518±0.200°.

16. The crystalline form B according to claim 15, wherein the X-ray powder diffraction pattern comprises characteristic peaks at 2θ angles of 7.333°, 11.034°, 12.635°, 13.534°, 13.855°, 14.350°, 16.116°, 17.483°, 17.861°, 18.764°, 19.658°, 19.915°, 20.651°, 21.157°, 23.449°, 23.806°, 24.154°, 24.702°, 26.884°, 27.518°, and 31.071°.

17. The crystalline form B according to any of claims 13 to 16, wherein the XRPD pattern is substantially as shown in FIG. 5.

18. The crystalline form B according to any of claims 13 to 16, wherein the differential scanning calorimetry profile has an onset value of an endothermic peak at 193.63±3.0°C.

19. The crystalline form B according to claim 18, wherein the DSC profile is as shown in FIG. 6.

20. The crystalline form B according to any of claims 13 to 16, wherein the thermogravimetric analysis profile of the crystalline form B shows a weight loss of 0.867% at 92±3°C.

21. The crystalline form B according to claim 20, wherein the TGA profile is as shown in FIG. 7.

22. Crystalline form C of the compound of formula (III), wherein n is selected from the group consisting of 0.8, 0.9, 1.0, 1.1 and 1.2, and the crystalline form C has an X-ray powder diffraction pattern comprising characteristic peaks at 2θ angles of 13.637±0.200°, 14.138±0.200°, 17.076±0.200°, and 24.866±0.200°,

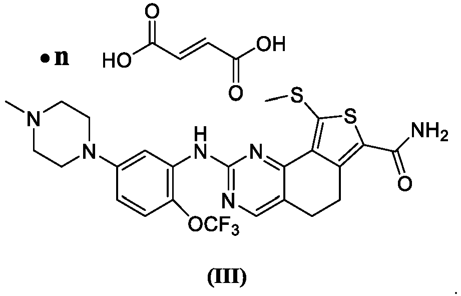

23. The crystalline form C according to claim 22, wherein the X-ray powder diffraction pattern comprises characteristic peaks at 2θ angles of 11.412±0.200°, 13.637±0.200°, 14.138±0.200°, 17.076±0.200°, 19.310±0.200°, 23.654±0.200°, 24.214±0.200°, and 24.866±0.200°.

24. The crystalline form C according to claim 23, wherein the X-ray powder diffraction pattern comprises characteristic peaks at 2θ angles of 11.412±0.200°, 13.637±0.200°, 14.138±0.200°, 15.555±0.200°, 17.076±0.200°, 18.016±0.200°, 18.907±0.200°, 19.310±0.200°, 20.811±0.200°, 23.654±0.200°, 24.214±0.200°, and 24.866±0.200°.

25. The crystalline form C according to claim 24, wherein the X-ray powder diffraction pattern comprises characteristic peaks at 2θ angles of 5.677°, 7.086°, 11.412°, 11.627°, 13.200°, 13.429°, 13.637°, 14.138°, 14.499°, 15.167°, 15.555°, 15.763°, 17.076°, 17.272°, 18.016°, 18.171°, 18.614°, 18.907°, 19.310°, 19.656°, 20.016°, 20.811°, 21.765°, 22.748°, 23.443°, 23.654°, 23.880°, 24.214°, 24.577°, 24.866°, 25.987°, 26.493°, 27.011°, and 27.537°.

26. The crystalline form C according to any of claims 22 to 25, wherein the XRPD pattern is substantially as shown in FIG. 9.

27. The crystalline form C according to any of claims 22 to 25, wherein the differential scanning calorimetry profile has an onset value of an endothermic peak at 237.14±3.0°C.

28. The crystalline form C according to claim 27, wherein the DSC profile is as shown in FIG. 10.

29. The crystalline form C according to any of claims 22 to 25, wherein the thermogravimetric analysis profile of the crystalline form C shows a weight loss of 0.469% at 100±3°C.

30. The crystalline form C according to claim 29, wherein the TGA profile is as shown in FIG. 11.

31. Crystalline form D of the compound of formula (IV), wherein p is selected from 0.8, 0.9, 1.0, 1.1 and 1.2, and the X-ray powder diffraction pattern comprises characteristic peaks at 2θ angles of 6.859±0.200°, 8.784±0.200°, 16.420±0.200°, and 18.965±0.200°,

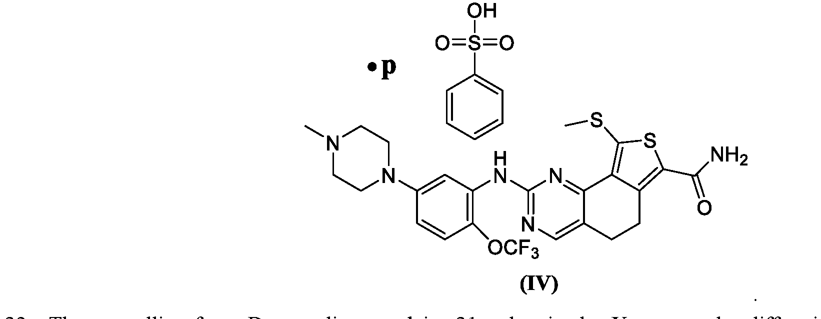

32. The crystalline form D according to claim 31, wherein the X-ray powder diffraction pattern comprises characteristic peaks at 2θ angles of 6.859±0.200°, 8.784±0.200°, 10.922±0.200°, 13.238±0.200°, 16.420±0.200°, 18.965±0.200°, 20.400±0.200°, and 24.069±0.200°.

33. The crystalline form D according to claim 32, wherein the X-ray powder diffraction pattern comprises characteristic peaks at 2θ angles of 6.859±0.200°, 8.784±0.200°, 10.922±0.200°, 11.644±0.200°, 13.238±0.200°, 16.420±0.200°, 17.596±0.200°, 18.965±0.200°, 19.390±0.200°, 20.400±0.200°, 21.416±0.200°, and 24.069±0.200°.

34. The crystalline form D according to claim 33, wherein the X-ray powder diffraction pattern comprises characteristic peaks at 2θ angles of 6.859°, 8.784°, 10.922°, 11.644°, 13.238°, 13.741°, 16.420°, 16.720°, 17.596°, 17.930°, 18.965°, 19.390°, 20.400°, 21.416°, 22.630°, 23.473°, 24.069°, 24.832°, 26.587°, and 27.661°.

35. The crystalline form D according to any of claims 31 to 34, wherein the XRPD pattern is substantially as shown in FIG. 13.

36. The crystalline form D according to any of claims 31 to 34, wherein the differential scanning calorimetry profile has an onset value of an endothermic peak at 240.70±3.0°C.

37. The crystalline form D according to claim 36, wherein the DSC profile is as shown in FIG. 14.

38. The crystalline form D according to any of claims 31 to 34, wherein the thermogravimetric analysis profile shows a weight loss of 0.295% at 120±3°C.

39. The crystalline form D according to claim 38, wherein the TGA profile is as shown in FIG. 15.

40. The salt according to any of claims 1 to 3, the crystalline form A according to any of claims 4 to 12, the crystalline form B according to any of claims 13 to 21, the crystalline form C according to any of claims 22 to 30 or the crystalline form D according to any of claims 31 to 39 in the preparation of a drug for treating a solid tumor.

41. The use according to claim 40, wherein the drug for treating a solid tumor is a drug for treating colorectal cancer.
